(19) Europäisches Patentamt
European Patent Office
Office européen des brevets

(11) EP 3 795 142 B1

(12) **EUROPÄISCHE PATENTSCHRIFT**

(45) Veröffentlichungstag und Bekanntmachung des Hinweises auf die Patenterteilung:
**15.02.2023 Patentblatt 2023/07**

(21) Anmeldenummer: **20195803.0**

(22) Anmeldetag: **11.09.2020**

(51) Internationale Patentklassifikation (IPC):
***A61K 9/14*** *(1974.07)* ***A61K 33/10*** *(1974.07)*
***A61K 9/16*** *(1974.07)* ***A61K 9/00*** *(1968.09)*
***A61K 9/50*** *(1974.07)* ***A61K 9/19*** *(1995.01)*

(52) Gemeinsame Patentklassifikation (CPC):
**A61K 9/143; A61K 9/0053; A61K 9/0095; A61K 9/1611; A61K 9/1652; A61K 9/1694; A61K 9/19; A61K 9/5026; A61K 33/10**

(54) **PORÖSES MAGNESIUMOXIDCARBONAT ZUR VERWENDUNG ALS MITTEL ZUR BINDUNG VON PHOSPHATEN IM DÜNNDARM MIT DEM ZIEL DER REDUZIERUNG VON NICHTÜBERTRAGBAREN KRANKHEITEN**

POROUS MAGNESIUM OXIDE CARBONATE FOR USE AS A MEANS FOR BINDING PHOSPHATES IN THE SMALL INTESTINE WITH THE AIM OF REDUCING NON-TRANSMISSIBLE DISEASES

CARBONATE D’OXYDE DE MAGNÉSIUM POREUX DESTINÉ À L’UTILISATION EN TANT QU’AGENT DE LIAISON DES PHOSPHATES DANS LE PETIT INTESTINAL DANS L’OBJECTIF DE RÉDUIRE LES MALADIES NON TRANSMISSIBLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.09.2019 DE 102019213938**

(43) Veröffentlichungstag der Anmeldung:
**24.03.2021 Patentblatt 2021/12**

(73) Patentinhaber: **FIM Biotech GmbH**
**17098 Friedland (DE)**

(72) Erfinder: **Krüger, Gerd**
**12621 Berlin, (DE)**

(74) Vertreter: **Maikowski & Ninnemann**
**Patentanwälte Partnerschaft mbB**
**Postfach 15 09 20**
**10671 Berlin (DE)**

(56) Entgegenhaltungen:
**DE-A1-102012 209 411**

- **L.A. HOLLINGBERY ET AL: "The thermal decomposition of huntite and hydromagnesite-A review", THERMOCHIMICA ACTA, Bd. 509, Nr. 1-2, 1. September 2010 (2010-09-01), Seiten 1-11, XP055538250, AMSTERDAM, NL. ISSN: 0040-6031, DOI: 10.1016/j.tca.2010.06.012**
- **HOLLINGBERY L A ET AL: "The thermal decomposition of natural mixtures of huntite and hydromagnesite", THERMOCHIMICA ACTA, ELSEVIER SCIENCE PUBLISHERS, AMSTERDAM, NL, Bd. 528, 3. November 2011 (2011-11-03), Seiten 45-52, XP028395198, ISSN: 0040-6031, DOI: 10.1016/J.TCA.2011.11.002 [gefunden am 2011-11-12]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 795 142 B1

**Beschreibung**

Die vorliegende Erfindung betrifft eine magnesiumhaltige Verbindung zur Verwendung gemäß Anspruch 1 zur Reduzierung von Nahrungsphosphaten im Dünndarm mit dem Ziel der Reduzierung von nichtübertragbaren Krankheiten, insbesondere zum Schutz der Nephrone (Untereinheit der Niere) vor Überlastung und der damit verbundenen Erhöhung des Friboblast Growth Factor 23.

**Beschreibung**

Foreman et al. ("Forecasting life expectancy, years of life lost, and all-cause and cause-specific mortality for 250 causes of death: reference and alternative scenarios for 2016-40 for 195 countries and territories") prognostizieren, dass sich von 2016 bis zum Jahr 2040 die Anzahl der Todesfälle durch nichtübertragbare Krankheiten (Non-communicable diseases NCDs) von 39,5 Mio. auf 60,9 Mio., d.h. von 72% auf 81% der gesamten Todesfälle, erhöhen wird. Das ist ein Zuwachs von insgesamt 54,4%. Die Weltbevölkerung wird sich in diesem Zeitraum lediglich um 17,8% von 7,3 Mrd. auf 8,6 Mrd. Menschen erhöhen. Die wichtigsten NCDs sollen sich wie folgt verändern: Krebserkrankungen +67%, kardiovaskuläre Erkrankungen +24%, Erkrankungen des Atmungssystems +48%, neurologische Erkrankungen +139%, davon Alzheimer +142%, Diabetes +107% und chronische Nierenerkrankungen +157%. Die Hauptursachen für diese Entwicklung werden wie folgt gewichtet: hoher Blutdruck, hoher Body-Mass-Index, hoher nüchtern Blutzucker, Rauchen, Alkoholmissbrauch, hoher Cholesterinspiegel, verringerte Nierenfunktion, mangelnde Qualität der Ernährung, geringes Geburtsgewicht, Bewegungsarmut und andere Faktoren.

Die Weltgesundheitsorganisation WHO hat verschiedene Programme aufgelegt, um insbesondere in den Entwicklungsländern die Gesundheitssituation zu verbessern. Die Bekämpfung der übertragbaren Krankheiten war erfolgreich und deren Anteil an den Todesursachen geht zurück. Gleichzeitig nehmen die nichtübertragbaren Krankheiten überproportional zu. Dabei spielt die Umstellung von einer traditionellen auf eine westlich orientierte Ernährung eine große Rolle.

In den entwickelten Ländern sind die NCDs das Hauptproblem. Trotz eines gut ausgebauten Gesundheitssystems nehmen die Krankheitsfälle nicht ab, sondern zu. Gleichzeitig hat sich ein neues Phänomen entwickelt, das als "Epidemiologie der Multimorbiditäten" bezeichnet werden kann. Das gleichzeitige Auftreten verschiedener chronischer Erkrankungen, die auch noch mit weiteren Komorbiditäten wie körperlichen und mentalen Gesundheitsproblemen verbunden sind, stellen die Gesundheitssysteme weltweit vor großen Herausforderungen, da diese Systeme bisher nur auf einzelne Erkrankungen ausgerichtet waren. Verschiedene Forschungsgruppen haben sich mit dieser Problematik auseinandergesetzt.

In der Studie von Barnet et al. ("Epidemiology of multimorbodity and implications for health care, research, and medical education: a cross-sectional study", Lancet, 2012, 380: 37-43) wurden in Schottland aus einer Datenbank mit 1,75 Mio. Personen, das entspricht etwa 30% der schottischen Bevölkerung, Daten zu 40 Krankheiten nach Geschlecht, Alter und sozioökonomischen Status ausgewertet. Danach wiesen 42,2% aller Patienten eine oder mehrere Morbiditäten auf und 23,2% waren multimorbid. Die Prävalenz der Multimorbidität nahm mit dem Alter erheblich zu, die absolute Menge der Multimorbiden lag jedoch in der Altersklasse unterhalb der 65 Jahren. Der Beginn der Multimorbidität war bei Menschen mit einem geringen sozioökonomischen Status ca. 10 bis 15 Jahre früher als bei der wohlhabendsten Gruppe. Körperliche und mentale Komorbiditäten zeigten die gleiche Tendenz.

Die Studie von S. Lichter et al. (Lifetime risk and multimorbidity of non-communicable diseases and disease-free life expectancy in the general population: A population-based cohort study; PLos Med, 2019, 16) kommt zu ähnlichen Ergebnissen. Dabei wurde insbesondere untersucht, wie die Risikofaktoren Rauchen, Bluthochdruck und Übergewicht die Inzidenz zu den NCDs, hier Schlaganfall, Herzerkrankungen, Diabetes, Chronische Lungenkrankheiten, Krebs und neurogenerative Erkrankungen, beeinflussen. Die Studie legt nahe, dass in einer westeuropäischen Gemeinschaft 90% der Personen älter als 45 Jahren in der verbleibenden Lebenszeit eine und anschließend mehrere NCDs entwickeln. Das Fehlen der 3 Risikofaktoren ist zwar mit einer Kompression der Morbidität der nichtübertragbaren Krankheiten von ca. 9 Jahren verbunden, aber die Frage bleibt, welche weiteren Faktoren zur Entwicklung von NCDs beitragen.

Das Vorhandensein von Multiorganerkrankungen stellt die Medizin vor besonderen Herausforderungen. Im Kontext multifaktorieller und komplexer Krankheitsbilder stellt sich die grundlegende Frage, ob es endogene systemische Ursachen gibt, die gleichzeitig eine Vielzahl von Organen und Systemen beeinflussen und verändern.

In Zoccali et al. (The systemic nature of CKD, Nat Rev Nephrol., 2017, 13: 344-358) wurde eine ausführliche Bilanz gezogen hinsichtlich der Forschungsarbeiten zur systemischen Natur der Nierenerkrankungen. Die bisher vorliegenden Ergebnisse bestätigen, dass eine nachlassende Nierenfunktion und die damit verbundenen metabolischen und endokrinen Veränderungen einen außerordentlichen Einfluss auf die Funktionsfähigkeit aller Organe ausüben. Für die Entwicklung von Arzneimitteln zum gezielten Eingriff in die komplexen Multiorgan-Beziehungen ist die Erforschung entsprechender endokriner Faktoren und Markern besonders wichtig. Wird jedoch ein präventiver Ansatz verfolgt, dann stehen die Ursachen der Nierenveränderung, deren Folgen und die Möglichkeiten der Vermeidung im Mittelpunkt des Interesses.

Das Wissen der Nephrologie hat sich in den vergangenen 20 Jahren stürmisch entwickelt.

In Makoto Kuro-o et al. (Mutation of the mouse klotho gene leads to a syndrome resembling ageing, Nature, 1997, 390:45-51) wird die Entdeckung des Klotho-Gens beschrieben. Die untersuchte Maus wies einen Phänotyp auf, die dem vorzeitigen Altern des Menschen, wie bei der fortschreitenden Nierenerkrankung üblich, ähnelten. Zu den altersbedingten Phänotypen gehörten eine verkürzte Lebensdauer, die Veränderung mehrerer Organe (Gonaden, Thymus, Haut u.s.w.), Gefäßverkalkung, Herzhypertrophie, Osteopenie, Sarkopenie, Wahrnehmungsstörungen, Hörstörung und Gebrechlichkeit. Also alles Phänotypen, die auch beim Menschen beobachtet werden können. Diese Symptome konnten alle auf die verminderte Expression nur eines Gens, dem Klotho-Gen, zurückgeführt werden. Es wird hauptsächlich aus den Zellen des Nierentubulus exprimiert. 2006 wurde die Klotho-Proteinfunktion als Rezeptor für den Fibroblasten-Wachstumsfaktor 23 (FGF23) identifiziert (Kurosu et al., J.Biol. Chem., 2006, 281: 6120-3).

In einem Review von Makato Kuru-o et al. (Molecular Mechanisms Underlying Accelerated Aging by Defects in the FGF23-Klotho System, Int. J. Nephrology, 2018) werden die wichtigsten Forschungsergebnisse bis Ende 2017 zusammengefasst. Folgendes Fazit wurde gezogen: Die Entdeckung des Klotho-Gens und seiner Funktion als obligater Korezeptor für FGF23 zeigte eine neue endokrine Achse auf, die für die Aufrechterhaltung der Phosphathomöostase unabdingbar ist. Mäuse mit Klotho- oder FGF23-Mangel altern vorzeitig. Durch Phosphatrestriktion konnten sie von alterungsähnlichen Phänotypen befreit werden. Das lässt den Schluss zu, dass das Phosphat das Altern beschleunigt. Phosphat übt seine Toxizität aus, wenn es an Kalzium bindet und zu Kalziumphosphat (CaPi) wird. CaPi bindet im Blut an das Serumprotein Fetuin-A und bildet kolloidale Partikel, die als CPP bezeichnet werden. CPP steigt mit dem Alter und dem Serumphosphatspiegel an und kann Gefäßendothelschäden, Verkalkung der glatten Muskulatur und angeborene Immunantworten hervorrufen, was zu Arteriosklerose und chronischer nichtinfektiöser Entzündung führt. In der tubulären Nierenflüssigkeit kann CPP auftreten, wenn die Phosphatausscheidung pro Nephron durch FGF23 erhöht wird. Die CPP induzieren dadurch Nierentubulusschäden und interstitielle Fibrosen, die die Nierenalterung beschleunigen und die Erkrankungen verschlimmern.

Edward R. Smith et al. (Biochemical transformation of calciprotein particles in uraemia, Bone. 2018 May; 110:355-367) haben neue Erkenntnisse zum Thema CPP und Urämie veröffentlicht. Sie konnten nachweisen, dass in einem urämischen Blutserum die amorphen CPP-I in einen kristallinen Zustand CPP-II wechseln. Die physiochemischen Veränderungen zu gepackten lamellenartigen kristallinen Hydroxylapatit gingen einher mit einer Anreicherung von verschiedensten Proteinen, Komplementfaktoren, Fettsäuren, DNA-Fragmente, kleine RNA, Endotoxine, Urämietoxine wie Indoxylsulfate u.s.w. Dadurch wirken diese CPP-II wie hochaktive endogene Pathogene. Eine Ingenuity-Pathway-Analyse wurde verwendet, um zelluläre Funktionen und Krankheitsprozesse zu identifizieren, die mit der Anreicherung von Proteinen in den CPP-II assoziiert waren (P<0,01). Dazu gehören: Amyloidose, Alzheimer, Demenz, Blutgerinnsel, chronische Entzündungen, Fibringerinnsel, Koagulationen des Blutes, Thrombosen, rheumatische Erkrankungen, systemische Autoimmunsyndrome, Herzinfarkt, Krebserkrankungen, Lebererkrankungen, Nierenerkrankungen u.a. Diese Aufzählung macht deutlich, dass die CPP-II systemische Wirkungen zeigen. Sie sind der Link zu den Multimorbiditäten.

In Yutaka Miura et al. (Identification and quantification of plasma calciprotein particles with distinct physical properties in patiens with chronic kidney disease, Scientific Reports, 2018, 8) wurden neben der Herausarbeitung einer Analysenmethode zur Identifizierung der CPP die Anzahl der CPP in Abhängigkeit vom Serumphosphatspiegel, dem Produkt aus Serum Ca x P und der CKD-Stufe ermittelt. Erhöhen sich die Parameter, dann erhöht sich auch die Anzahl der CPP. Bei einem Serumphosphatspiegel von 2,5 mg/dl bilden sich praktisch keine CPP. Dies macht die Bedeutung eines niedrigen Serumphosphatspiegels besonders deutlich.

Pavic et al. (Secreted Klotho and FGF23 in chronic kidney disease Stage 1 to 5: a sequence suggested from a cross-sectional study, Nephrol. Dial. Transplant, 2013, 28:352-359) beschreibt das Verhalten der wichtigsten Schlüsselregulatoren wie Klotho, FGF23, Parathormon und 1,25 Vitamin D3 besonders deutlich. Dazu wurden Patienten aus allen CKD-Stufen im Vergleich zu einer gesunden Kontrollgruppe untersucht und die Ergebnisse über die eGFR aufgetragen. Das Fazit ist eindeutig: das lösliche Klotho und 1,25 Vitamin D3 verringern sich und FGF23 erhöht sich bereits in einer sehr frühen CKD-Stufe, wobei sich das Parathormon erst in einer bereits ausgeprägten CKD-Stufe erhöht.

Die Veränderung dieser Schlüsselregulatoren durch chronische Nierenerkrankungen oder Alterungsprozesse sind Sekundärfolgen, die primär mit einer Erhöhung der Phosphatausscheidung pro Nephron verbunden sind. Es gibt prinzipiell zwei Wege die Phosphatausscheidung in den Urin zu erhöhen: Eine besteht darin, das Volumen des ultrafiltrierten Blutplasmas durch Vergrößerung der glomerulären Filtrationsrate (GFR) der Niere durch eine Hypertrophie der Glomeruli (Hyperfiltration) zu erhöhen. Zum anderen kann die fraktionierte Phosphatausscheidung (FEp, 24h Urinphosphat) erhöht werden. Im Gegensatz zu gesunden Menschen mit einer hohen Nierenreserve sind CKD-Patienten nicht immer in der Lage, die GFR zu erhöhen. Sie sind aber in der Lage, durch Erhöhung der FEp das Phosphatgleichgewicht aufrechtzuerhalten. Der Anstieg der FEp ist in erster Linie auf einem Anstieg des Serum-FGF23 im Frühstadium der CKD zurückzuführen. Deshalb kann FGF23 als Biomarker für die Phosphatausscheidung pro Nephron verwendet werden.

Speer et al. (A single preoperativ FGF23 measurement is a strong predictor of outcome in patients undergoing elective cardiac surgery: a prospective observational study, Crit Care, 2015 19:190) konnte nachweisen, dass das Hazard Ratio (HR) für verschiedene Krankheiten ab einem cFGF23-Wert von 64 RU/ml stark ansteigt. Dies gilt auch für die CKD. Damit ist ein Grenzwert gefunden worden, ab dem sich die fraktionierte Phosphatausscheidung und damit verbunden das HR erhöht.

Das humane Mikrobiom ist Gegenstand vieler Forschungsarbeiten (Al Khodor et al. "Gut microbiome and kidney disease: a bidirectional relationship" Pediatr Nephrol, 2017, 32: 921-931). Die CKD ist oft mit einer Darmdysbiose verbunden, die durch eine veränderte Mikrobenzusammensetzung gekennzeichnet ist. Insbesondere die Anzahl pathogener Mikroben ist erhöht. Dafür gibt es verschiedene Ursachen. Erhöht sich FGF23 auf Grund einer erhöhten fraktionierten Phosphatausscheidung, wird das 1,25 Vitamin D3 (Calcitriol) heruntergeregelt, um die Phosphataufnahme im Dünndarm zu reduzieren und die Ausscheidung in der Niere zu erhöhen. Das hat auch Auswirkungen auf die intestinale alkalische Phosphatase und den intestinalen Tight Junctions. Auf der einen Seite wandern Polyphosphate in den Dickdarm ein und verändern das Mikrobiom, auf der anderen Seite kommt es zu einer erhöhten Permeabilität und Translokation von bakteriellen Produkten wie die Endotoxine. Durch eine erhöhte Fermentation von solchen Aminosäuren wie Tyrosin und Tryptophan im Dickdarm werden insbesondere p-Cresole und Indole generiert, die in der Leber zu p-Cresylsulfat und Indoxylsulfat metabolisiert werden. Diese Toxine werden von der Niere ausgeschieden, können aber dabei die Renalfunktionen beeinträchtigen und eine CKD begünstigen. Deshalb werden sie als Urämietoxine bezeichnet. Ist die Nierenleistung vermindert, dann können sie sich im Blut anreichern. Die Vielzahl der bakteriellen Produkte, die Urämietoxine und die sich bildenden kristallinen CPP-II erzeugen ein Entzündungsgeschehen im Körper, das durch das Akute-Phase-Protein hsCRP detektiert werden kann. Die Grundlage für die Herausbildung von Multimorbitäten ist gelegt. Die Niereninsuffizienz wird weiter gesteigert und damit die negative Rückkopplung zum Darm über das 1,25 Vitamin D3. Dieser Vicious Circle muss rechtzeitig unterbrochen werden.

Die Nierenleistung wird von der Anzahl intakter Nephrone bestimmt. In Puelles et al. (Glomerular number and size variability and risk for kidney disease, Curr. Opin. Nephrol. Hypertens., 2011, 20:7-15) wurde an Hand mehrerer menschlicher Populationen gezeigt, dass sich die quantitative Mikroanatomie der Niere erheblich unterscheidet: Die Anzahl der Nephrone variiert um bis das 13-fache, das mittlere Nephronvolumen bis zum 7-fachen und das Volumen der Nephrone in den einzelnen Nieren bis zum 8-fachen. Die Anzahl, Größe und Größenverteilung der Nephrone korrelieren mit den Risikofaktoren für Nierenerkrankungen, darunter Geburtsgewicht, Alter, Rasse, Körpergröße und Bluthochdruck.

Eine besondere Stellung nimmt das Geburtsgewicht ein. Hughson et al. (Glomerular number and size in autopsy kidneys: The relationship to birth weight, Kindney Int., 2003, 63:2113-22) stellten fest, dass das Geburtsgewicht eine starke Determinante für die Anzahl der Nephrone und deren Größe in der postnatalen Niere ist. Die Ergebnisse stützen die Hypothese, dass das geringe Geburtsgewicht (LBW) durch die Beeinträchtigung der Nephronentwicklung ein Risikofaktor für spätere Nierenerkrankungen und Hypertonie im Erwachsenenalter ist. Gurusinghe et al. (Developmental Origins and Nephron Endowment in Hypertension, Front Pediatr, 2017, 5: 151) bestätigen, dass an Hand zahlreicher Tier- und Humanstudien der umgekehrte Zusammenhang zwischen dem Blutdruck und der Anzahl der Nephrone nachgewiesen wurde. Diese Erkenntnis hat aus ihrer Sicht sowohl Auswirkungen auf die Behandlung der Hypertonie als auch auf die Vermeidung nichtübertragbarer Krankheiten (NCDs).

Haut et al. (Renal toxicity of phosphate in rats, Kidney Int., 1980, 17: 722-31) stellten fest, dass sich bei einer Phosphatausscheidung von >1$\mu$g/Tag und Nephron histologische Veränderungen im Nephron zeigen. Dazu gehörten interstitielle Ödeme, Fibrosen, tubuläre Atrophien und Dilatationen sowie Kalzifikationen. Diese Ergebnisse weisen darauf hin, dass der Phosphatausscheidung pro Nephron und Tag die größte Aufmerksamkeit geschenkt werden muss.

Die westliche Ernährungsweise ist gekennzeichnet durch einen hohen Phosphatgehalt. Insbesondere die industriell verarbeitete Nahrung enthält oft phosphathaltige Lebensmittelzusatzstoffe, um die Produkte haltbar zu machen oder die Konsistenz zu beeinflussen. Dies gilt auch für viele Getränke. Dem Verbraucher ist nicht bewusst, dass er durch die Einnahme dieser Nahrungsmittel einem Risiko ausgesetzt ist. Insbesondere eine Gruppe trägt dabei ein großes Risiko, Schäden an den Nephronen der Niere zu erleiden: Personen mit einem Geburtsgewicht von <3 kg. Etwa 10% der Weltbevölkerung gehört zu dieser Gruppe, also ca. 800 Mio. Menschen. Es ist davon auszugehen, dass ein Großteil dieser Menschen NCDs im Verlauf ihres Lebens entwickeln werden.

Hughson et al. (Glomerular number and size in autopsy kidneys: The relationship to birth weight, Kindney Int., 2003, 63:2113-22) bestimmten die Phosphatausscheidung pro Nephron im $\mu$g/Tag in Abhängigkeit vom Geburtsgewicht, Alter und 1400mg/Tag Phosphatabsorption. So hat eine 20jährige Person bei einem Geburtsgewicht von 3kg die gleiche Anzahl von Nephronen (1,5 Mio.) wie ein 50jähriger mit einem Geburtsgewicht von 3,5kg. Es wurde bereits festgestellt, dass eine Phosphatausscheidung pro Nephron von >1$\mu$g/Tag einen kritischen Wert darstellt. Ab diesem Wert ist mit Verletzungen der Nephrone zu rechnen. Bei einer angenommenen Phosphatabsorption von 1400mg/Tag wird dieser Wert schon in sehr jungen Jahren zwischen 25 und 35 erreicht. Bei einem normalen Geburtsgewicht geschieht das erst mit ca. 65 Jahren. Diese Modellrechnungen für gesunde Personen sollen verdeutlichen, dass eine Phosphataufnahme, die der Anzahl der Nephrone gerecht wird, der Schlüssel zur Gesundheit ist.

Die Phosphataufnahme mit der Nahrung ist kaum quantifizierbar. Deshalb ist der Biomarker cFGF23 das Mittel der Wahl. Ist der Wert größer als 64 RU/ml, dann sollte gehandelt werden. Eine Reduzierung von cFGF23 ist nur über die Verringerung der Phosphatabsorption möglich. Folgende pathogene Kaskade wird damit unterbunden: keine Überlastung der Nephrone, keine Beschädigungen, keine erhöhte fraktionierte Phosphatausscheidung; keine Erhöhung von FGF23, keine Reduzierung von 1,25 Vitamin D3 und Klotho; keine negative Beeinflussung des Mikrobioms, keine Erhöhung pathogener Keime, keine erhöhte Permeabilität und Translokation von bakteriellen Produkten wie die Endotoxine, keine erhöhte Bildung von p-Cresole und Indole; keine Herausbildung von kristallinen CPP-II, da kein urämisches Milieu vorhanden ist; keine Beschädigungen von Gefäßen und Organen durch die CPP-II, da nicht vorhanden; keine erhöhte Entzündungslage; keine Trigger für die Herausbildung von NCDs vorhanden, die mit den CPP-II im direkten Zusammenhang stehen.

Phosphatbinder werden üblicherweise bei einer Hyperphosphatämie (Serumphosphatspiegel >5mg/dl) eingesetzt, die erst in einer sehr späten Phase der Nierenerkrankung (GFR-Stufe 4 und 5) entsteht. Suetonia C. Palmer et al. (Phosphate-Binding Agents in Adults With CKD: A Network Meta-analysis of Randomized Trials, Am J Kidney Dis. 2016 Nov;68(5):691-702) haben mit ihrer Arbeit nachgewiesen, dass der Einsatz von Phosphatbindern in dieser Phase keine Reduktion der Mortalität im Vergleich zu einem Placebo erreicht. Vielmehr wurden viele Nebenwirkungen wie Durchfall, Verstopfung, Übelkeit und Erbrechen verzeichnet. Diese waren bei den einzelnen Phosphatbindern unterschiedlich ausgeprägt, aber keiner war frei von Nebenwirkungen. DE 10 2012 209 411 beschreibt die Verwendung eines Wechsellagerungstonminerals aus Montmorillonit und Illit/Muskovit als Phosphatbinder.

Alle derzeitigen Phosphatbinder benötigen die Magensäure entweder zum Auflösen der Verbindungen und damit Freisetzung der Kationen oder zur Aktivierung von Molekülen für die spätere Bindung der Phosphate. Damit wird in den Säurehaushalt und somit in die Verdauungsarbeit des Magens stark eingegriffen, was teilweise die Nebenwirkungen verursacht.

Der vorliegenden Erfindung liegt daher das Problem zu Grunde, eine Verbindung bzw. Zusammensetzung zur Reduzierung des Phosphatgehaltes in Menschen zur Verfügung zu stellen, welche die oben genannten Nachteile nicht aufweist.

Die Erfindung hat sich des Weiteren das Ziel gesetzt ein Mittel und ein Verfahren zur dessen Herstellung zur Verfügung zu stellen, um die Beschädigung der Nephrone und damit den Anstieg von FGF23 zu verhindern. Das soll durch die Bindung von Nahrungsphosphaten im Dünndarm erreicht werden, ohne dass Magen und Darm negativ beeinflusst werden.

Diese Aufgabe wurde durch einen porösen Komplex aus Magnesiumoxid/-carbonat mit den Merkmalen des Anspruchs 1 gelöst.

Demnach wird ein poröses Magnesiumoxidcarbonat zur Verwendung als Mittel zur Bindung von Phosphaten im Dünndarm zur Reduzierung von nichtübertragbaren Krankheiten bereitgestellt, welches gekennzeichnet ist durch:

- eine mittlere Teilchengröße zwischen 0,5 und 3μm, bevorzugt zwischen 0,8 und 2μm, insbesondere bevorzugt 1μm;
- Mesoporen mit einen mittleren Porendurchmesser zwischen 3,5 und 6,5nm und Makroporen mit einem mittleren Porendurchmesser zwischen 50 und 200nm, wobei die spezifische Oberfläche der Mesoporen zwischen 70 und 90 $m^2/g$ und die spezifische Oberfläche der Makroporen zwischen 10 und 14 $m^2/g$ beträgt, und
- einen Absorptionsindex $AB_{IDX}$ für die Bindung von Phosphat zwischen 30 bis 40, bevorzugt von 32 bis 38, insbesondere bevorzugt von 35.

In einer Ausführungsform weist das poröse Magnesiumoxidcarbonat eine maximale Phosphatbindekapazität $Q_{max}$ zwischen 3500 und 4000 mg/g Absorber, bevorzugt zwischen 3700 und 3900 mg/g Absorber auf. $Q_{max}$ als maximale Phosphatbindekapazität wird aus dem Langmuir-Isothermen-Modell unter Nutzung von in vitro Versuchen bestimmten Absorptionswerten bei ca. pH 7 berechnet. Es ist ein theoretischer Wert, der sich bei einer sehr hohen Phosphatbelastung pro Menge Absorber ergibt.

Das vorliegende poröse Magnesiumoxidcarbonat wird bevorzugt aus einem Hydromagnesiten hergestellt, der nach der Zerkleinerung einer thermischen Behandlung (Kalzinierung) bei einer Temperatur zwischen 400 und 600°C, bevorzugt zwischen 450 und 550°C, insbesondere bevorzugt bei 500°C über einen Zeitraum von 40 bis 80 min, bevorzugt 50 bis 70 min, insbesondere bevorzugt 60 min unterzogen wird.

Die thermische Behandlung des Hydromagnesits bewirkt eine Dehydration, eine Dehydroxylierung und eine Dekarbonisierung. Dadurch bildete sich in überraschenderweise ein stabiler Mg-Komplex mit einer porösen Struktur heraus, der sich gegenüber dem Ausgangsmaterial durch eine wesentlich verbesserte Phosphatbindekapazität unterscheidet. Auch weist der poröse Mg-Komplex im Vergleich zu den bisher typischerweise verwendeten Phosphatbindern seine maximale Phosphatbindung als Feststoff in einem basischen Milieu auf. Eine Auflösung bzw. Aktivierung der Verbindung im Magen ist nicht notwendig und vermeidet dadurch deren unerwünschten Nebenwirkungen.

Wie bereits angedeutet wird der Hydromagnesit vor der thermischen Behandlung einer Mikronisierung unterzogen. Dabei wird er zunächst mit Hilfe einer Dampfstrahlmühle auf eine mittlere Teilchengröße zwischen 0,5 und 3μm, bevorzugt zwischen 0,8 und 2μm, insbesondere bevorzugt 1μm zerkleinert. Wird anschließend die oben beschriebene thermische Behandlung durchgeführt, erhöht sich die Phosphatbindekapazität nochmals um das mehrfache.

Die Verbindung der einzelnen Prozessschritte erzeugt im porösen Mg-Komplex eine Porenstruktur, die optimal auf die Phosphatbindung abgestimmt ist. Mit Hilfe der Quecksilberporosimetrie wurden die Porengrößenverteilung und die spezifische Oberfläche bestimmt.

Mesoporen sind definitionsgemäß Poren mit einem Durchmesser von 3 bis 20nm und Makroporen von 20 bis 500nm. Größere Poren wurden als Zwischenkornvolumen gewertet und nicht berücksichtigt. Aus dem intrudierten Volumen der Mesoporen $iV_{MEP}$ und der Makroporen $iV_{MAP}$ sowie den spezifischen Oberflächen der Mesoporen $sA_{MEP}$ und der Makroporen $sA_{MAK}$ konnten volumenbezogene Oberflächen wie folgt abgeleitet werden:

$$AV_{MEP} = sA_{MEP}/iV_{MEP}$$

und

$$AV_{MAP} = sA_{MAP}/iV_{MAP}$$

abgeleitet werden.

Auf dieser Grundlage konnte ein Absorptionsindex $AB_{IDX}$ wie folgt bestimmt werden:

$$AB_{IDX} = AV_{MEP} / AV_{MAP}$$

Die Höhe von $AB_{IDX}$ ist für die Phosphatbindung eine entscheidende Größe. Wie oben erwähnt weist der erfindungsgemäße poröse Mg-Komplex einen $AB_{IDX}$ von 30 bis 40, bevorzugt von 32 bis 38 und insbesondere bevorzugt von 35 auf. Dies kann nur erreicht werden, wenn die volumenbezogenen Oberflächen der Mesoporen wesentlich größer sind als die der Makroporen.

Der mittlere Porendurchmesser der Mesoporen beträgt zwischen 3,5 und 6,5nm, bevorzugt zwischen 4 und 6nm und insbesondere bevorzugt 5nm, und der mittlere Porendurchmesser der Makroporen beträgt zwischen 50 und 200nm, bevorzugt zwischen 100 und 150nm und insbesondere bevorzugt 125nm.

Für eine optimale Phosphatbindung beträgt die spezifische Oberfläche in $m^2/g$ der Mesoporen zwischen 70 und 90 $m^2/g$, bevorzugt zwischen 75 und 85 $m^2/g$ und insbesondere bevorzugt 80 $m^2/g$, die der Makroporen zwischen 10 und 14 $m^2/g$, bevorzugt zwischen 11 und 13 $m^2/g$ und insbesondere bevorzugt 12 $m^2/g$.

Hydromagnesite sind Teil eines tertiären Systems aus Magnesit ($MgCOs$), Brucit ($Mg(OH)_2$) und Wasser ($H_2O$). Mit Hilfe eines Phasendiagramms für $MgO$-$CO_2$-$H_2O$ ist leicht zu erkennen, unter welchen Druckbedingungen welche feste Phase natürlich entsteht (W.B. White, Environmental Geology, 1997, 30: 46-58). In der Veröffentlichung EP 2 692 691 A1 wird ein technischer Herstellungsprozess für Magnesite und Hydromagnesite ausführlich behandelt.

Der Hydromagnesit hat die chemische Formel $Mg_5(CO_3)_4(OH)_2{*}4H_2O$. Eine ähnliche Zusammensetzung hat der Dypingit, $Mg_5(CO_3)_4(OH)_2{*}5H_2O$, der lediglich ein Wassermolekül mehr besitzt. Das Wort Hydromagnesit soll alle Arten umfassen, die eine solche Struktur entsprechend des tertiären Systems besitzen.

Thermogravimetrische Analysen zeigen, dass Hydromagnesite bis zu 60% ihres Gewichtes durch Wärmeeinwirkung verlieren können. Deshalb sind sie besonders prädestiniert für die Herausbildung einer hohen inneren Oberfläche.

Künstlich hergestellte Hydromagnesite können so produziert werden, dass sie den Anforderungen der internationalen Pharmakopöen entsprechen. Sie werden als Abführmittel, als mildes Magenmittel und als Viskositätsregler in der Pharmaindustrie eingesetzt. Für die erfindungsgemäße Herstellung des porösen Mg-Komplexes wird ein pharmagerechter Hydromagnesit verwendet. Die mittlere Teilchengröße $X_{50}$ in $\mu$m des Ausgangsstoffes Hydromagnesit sollte zwischen 20 und 40, bevorzugt zwischen 25 und 35 und besonders bevorzugt bei 30 liegen.

Das vorliegende poröse Magnesiumoxidcarbonat wird in einem Verfahren mit den folgenden Schritten hergestellt:

a) Zerkleinerung von Hydromagnesit auf eine mittlere Teilchengröße zwischen 0,5 und 3$\mu$m, bevorzugt zwischen 0,8 und 2$\mu$m, insbesondere bevorzugt 1$\mu$m;
und
b) thermische Behandlung des zerkleinerten Hydromagnesit bei einer Temperatur zwischen 400 und 600°C, bevorzugt zwischen 450 und 550°C, insbesondere bevorzugt
bei 500°C über einen Zeitraum von 40 bis 80 min, bevorzugt 50 bis 70 min, insbesondere bevorzugt 60 min.

Die Zerkleinerung des Hydromagnesits als Ausgangsstoff mit einer mittleren Teilchengröße von ca. 30μm erfolgt mit einer Dampfstrahlmühle auf eine Endfeinheit von $X_{50}$ von ca. 1μm. Die sich anschließende thermische Behandlung des mechanisch aktivierten Hydromagnesiten wird in eine bevorzugten Ausführungsvariante bei ca. 500°C und für eine Zeitdauer von ca. 60min entweder in einer unbewegten dünnen Schicht (Hochtemperatur Reinraum-Wärmeschrank), in einer durch Rotation erzeugten bewegten Schicht (Drehrohrofen) oder in einer mechanisch oder pneumatisch erzeugten Wirbelschicht vorgenommen.

In einer weitergehenden Ausführungsform wird das nach der thermischen Behandlung erhaltene poröse (hydrophile) Magnesiumoxidcarbonat in einer inerten Gasatmosphäre z.B. Argon gelagert.

Gemäß dem vorliegenden Verfahren werden die Hydromagnesite somit einer mechanischen und thermischen Aktivierung unterzogen. Je nach Behandlungsschritt (d.h. Temperatur und Dauer) weist das poröse Mg-oxidcarbonat unterschiedliche maximale Phosphatbindekapazitäten $Q_{max}$ in mg/g auf. Der unbehandelte Hydromagnesit ist z.B. durch eine maximale Phosphatbindekapazität von ca. 119 mg/g gekennzeichnet, die durch die verschiedenen Behandlungsschritte zum Teil um ein Vielfaches gesteigert werden kann. So weist der lediglich zerkleinerte Hydromagnesit nur eine leichte Erhöhung der maximalen Phosphatbindekapazität von ca. 158 mg/g auf. Allerdings ist die Zerkleinerung für die Herausbildung der optimalen Porenstruktur von essentieller Bedeutung. Der anschließende Verfahrensschritt der thermischen Behandlung des vorab auf ca. 1μm zerkleinerten Hydromagnesiten erhöht die maximale Phosphatbindekapazität z.B. um über das 20fache auf ca. 3958 mg/g.

Zur weiteren Verwendung wird das poröse Magnesiumoxidcarbonat in einer Zellulosematrix stabilisiert. Entsprechend wird ein Granulat umfassend das poröse Magnesiumoxidcarbonat und eine Zellulosematrix, insbesondere eine Methyl-Zellulose-Matrix bereitgestellt.

In einer Ausführungsform weist das vorliegende Granulat ein Masseverhältnis von Zellulose und porösem Magnesiumoxidcarbonat zwischen 0,7:1,3, bevorzugt zwischen 0,9:1,1, insbesondere von 1:1 auf. Der Masseanteil des porösen Magnesiumoxidcarbonats als Phosphatabsorber im fertigen Granulat beträgt zwischen 40 und 60%, bevorzugt zwischen 45 und 55% und insbesondere bevorzugt bei 50%.

Das vorliegende Granulat weist einen mittleren Durchmesser zwischen 40 und 450μm, bevorzugt zwischen 250 bis 450μm, insbesondere bevorzugt zwischen 40 und 150μm auf.

In einer weiteren Ausführungsform ist das vorliegende Granulat mit einem magensaftresistenten Coating beschichtet. Als Coatingmateral werden bevorzugt Polymethacrylate, wie z.B. anionische Copolymere aus Methacrylsäure und Ethylacrylat (auch bekannt als Eudragit) verwendet. Das Masseverhältnis von Granulat und Coatingmaterial beträgt zwischen 30:1 und 20:1, bevorzugt zwischen 25:1 und 15:1, insbesondere bevorzugt von 10:1. Die magensaftresistenten Granulate können in Form von Kapseln, Gelen und Suspensionen vorliegen.

Das vorliegende Granulat wird in einem Verfahren mit den folgenden Schritten hergestellt:

a) Suspension des porösen Magnesiumoxidcarbonates in einer kolloidalen Zellulosedispersion, insbesondere kolloidalen Methylzellulosedispersion;
und
b) Granulierung der erhaltenen Suspension mittels Sprüh-Gefrier-Granulierung mit anschließender Gefriertrocknung.

In einer Variante des Verfahrens werden in Schritt a) weitere bioaktive Substanzen in die kolloidale Zellulosedispersion dispergiert.

In dem vorliegenden Verfahren wird das poröse Magnesiumoxidcarbonat bzw. der poröse Mg-Komplex in eine Zellulose-Matrix, z.B. Methylzellulose-Matrix, eingebettet. In einem zweiten Schritt erfolgt eine Sprüh-Gefrier-Granulierung mit anschließender Gefriertrocknung. Dazu wird Methylcellulose in Pharmaqualität in gereinigtem Wasser dispergiert. In die entstandene kolloidale Dispersion wird der poröse Mg-Komplex dispergiert. Das Masseverhältnis zwischen Methylcellulose und dem porösen Mg-Komplex beträgt bevorzugt ca. 1:1. Mittels Druckluft wird das Gemisch durch Düsen gepresst, deren Durchmesser zwischen 40 und 450 μm liegen können. Die kugelförmigen Tropfen fallen in ein Bad mit flüssigem Stickstoff (-196°C). Es entstehen Eis-Cellulose-Granulate mit dem eingebetteten porösen Mg-Komplex. Die gefrorenen Granulate werden einer Vakuum-Gefriertrocknung zugeführt. Das gefrorene Wasser wird durch Sublimation entfernt. Es entstehen Granulate aus einer trockenen Methyl-Cellulose-Matrix mit eingebetteten porösen Mg-Komplexen. Das poröse Granulat kann anschließend in einer inerten Gasatmosphäre wie z.B. Argon gelagert werden.

An die Granulierung kann sich die Beschichtung des Granulates mit dem magensaftresistenten Coating anschließen, die erfolgt bevorzugt in einem Wirbelschichtapparat erfolgt. Die magensaftresistente Beschichtung wird bevorzugt durch ein Wirbelschicht-Coating erzeugt. Dazu werden die porösen Granulate in einen Wirbelschichtapparat gegeben und eine Wirbelschicht, bevorzugt mit einem inerten Gas, erzeugt. Das flüssige Coating-Material, z.B. Eudragit L 100-55, wird eingesprüht und ummantelt die porösen Granulate.

Die gut rieselfähigen und magensaftresistenten Granulate können je nach Applikationsform in der Größe zwischen 40 und 450 μm hergestellt werden. Für die Abfüllung in Kapseln wären Granulate zwischen 250 bis 450 μm

vorteilhaft, in Flüssigkeiten dagegen Granulate zwischen 40 und 150μm.

Die magensaftresistenten Granulate aus einer Methyl-Cellulose-Matrix mit den eingebetteten porösen Mg-Komplexen können in Form von Kapseln oder in Flüssigkeiten oder Gelen verabreicht werden. In der Prävention und in den CKD-Stufen 1 bis 3 sind Verabreichungen in Flüssigkeiten und Gelen, ab der CKD-Stufe 4 in Kapseln vorzuziehen. Das fertige Granulat enthält ca. 50% des porösen Mg-Komplexes als Phosphatabsorber. Die notwendige Tagesdosis des Phosphatbinders in der Prävention und in den einzelnen CKD-Stufen bezieht sich auf den Absorber selbst. Danach sollte der Absorber 3 x täglich zur Mahlzeit in den CKD-Stufen 3-5 eingenommen werden. Zur Prävention und den CKD-Stufen 1 bis 2 ist eine einmalige Aufnahme zum Mittagessen ausreichend. Folgende Aufnahmemengen werden empfohlen: Prävention ca. 500mg, CKD-Stufe 1 ca. 900mg, CKD-Stufe 2 ca. 1400mg, CKD-Stufe 3 ca. 2000mg, CKD-Stufe 4 ca. 3000mg und CKD-Stufe 5 ca. 4000mg. Insgesamt ist eine individuelle Anpassung der Mengen für den Absorber notwendig und sie sind abhängig vom Ausgangs- und Zielwert für cFGF23 der einzelnen Personen.

Die vorliegende Erfindung wird anhand der folgenden Ausführungsbeispiele unter Bezugnahme auf die Figuren näher erläutert. Es zeigen:

- Figur 1 Vergleich der Pulver-Röntgendiffraktogramme verschiedener Aktivierungsstufen;
- Figur 2 Porenvolumen pro Gramm bezogen auf den Porendurchmesser für Meso- und Makroporen bei unterschiedlichen Aktivierungen von MG_18;
- Figur 3 Porenvolumen pro Gramm bezogen auf den Porendurchmesser für Meso- und Makroporen bei unterschiedlichen Aktivierungen von MG_18_DSM;
- Figur 4 Porenvolumen pro Gramm bezogen auf den Porendurchmesser für Meso- und Makroporen der wesentlichen Produktionsstufen;
- Figur 5 Zusammenhang zwischen der max. Phosphatbindekapazität $Q_{max}$ und dem Absorptionsindex $AB_{IDX}$;
- Figur 6 Phosphatabsorption verschiedener Aktivierungsstufen;
- Figur 7 Modellrechnungen zur Phosphat-Absorption in Abhängigkeit von der Absorber-Dosierung.

Die Bezeichnungen der einzelnen Proben unterliegen folgender Systematik:

| | | |
|---|---|---|
| 1. | MG_18 | Hydromagnesit, $X_{50}$ ca. 30μm |
| 2. | MG_18_K(500/60) | $X_{50}$ ca. 30μm, kalziniert 500°C, 60min |
| 3. | MG_18_K(850/60) | $X_{50}$ ca. 30μm, kalziniert 850°C, 60min |
| 4. | MG_18_DSM | Dampfstrahlmühle, $X_{50}$ ca. 1μm |
| 5. | MG_18_DSM_K(500/60) | $X_{50}$ ca. 1μm, kalziniert 500°C, 60min |
| 6. | MG_18_DSM_K(500/60) | $X_{50}$ ca. 1μm, kalziniert 850°C, 60min |
| 7. | MG_18_DSM_K(700/60) | $X_{50}$ ca. 1μm, kalziniert 700°C, 60min |
| 8. | MG_18_DSM_K(500-700/60) | $X_{50}$ ca. 1μm, kalziniert 500-700°C, 60min |

Vorliegend wurde ein Hydromagnesit auf seine Eigenschaften als Phosphatbinder untersucht, wobei der Hydromagnesit verschiedenen Behandlungsschritten ausgesetzt wurde.

In Figur 1 sind beispielhaft die Pulver-Röntgendiffraktogramme für den auf ca. 1μm zerkleinerten Hydromagnesiten MG_18_DSM und den bei 500°C und 850°C für jeweils 60min behandelten MG_18_DSM dargestellt. MG_18_DSM zeigt den für einen Hydromagnesiten typischen Verlauf und unterscheidet sich strukturell nicht vom unbehandelten MG_18. Wird dieses Material jedoch bei 500°C für 60min wärmebehandelt, dann kommt es strukturell zu dramatischen Veränderungen. Es entsteht ein meso- und makroporöser Komplex aus Magnesiumoxid/-carbonat. Wird die Behandlungstemperatur auf 850°C erhöht, dann entstehen makroporöse Magnesiumoxide. Mit Hilfe der Quecksilberporosimetrie konnte die Porenentstehung untersucht werden.

Figur 2 zeigt das auf die Masse bezogene Porenvolumen über den Porendurchmesser für verschiedene Aktivierungen von MG_18 aufgetragen. Bei einer Temperatur von 500°C und einer Behandlungsdauer von 60min entstehen Mesoporen zwischen 3 und 20nm, die insbesondere für die Phosphatbindung verantwortlich sind. Die spezifische Oberfläche sA in $m^2/g$ steigt von 12 auf 70. Wird MG_18 60min lang bei 850°C kalziniert, dann entstehen makroporöse Magnesiumoxide. Die Mesoporen verschwinden und die Phosphatbindung geht dabei stark zurück. Auch sA fällt wieder auf 15 $m^2/g$. Der Absorptionsindex $AB_{IDX}$ kann aus der Porengrößenverteilung berechnet werden und ist ein gutes Maß für die Phosphatbindekapazität. Gute Bezugspunkte sind auch die Zahlenpaare aus Masse bezogenem Porenvolumen [cc/g] und dem mittleren Porendurchmesser [QJ Pore nm] für die Meso- und Makroporen. Für die einzelnen Proben gilt: Nr.1 {Meso (-/-), Makro (0,3547/208)}; Nr.2 {Meso (0,2543/6,44), Makro (0,4433/194)} und Nr.3 {Meso (-/-), Makro (0,8359/159)}.

Figur 3 zeigt die nach der gleichen Systematik wie in Figur 2 aufgetragenen Kurvenverläufe für den auf ca.

1$\mu$m mittels Dampfstrahlmühle zerkleinerten Hydromagnesiten MG_18_DSM und dessen Aktivierungen. MG_18_DSM wurde wie MG_18 sowohl bei 500°C als auch bei 850°C für jeweils 60min kalziniert. Auch hier zeigen sich die gleichen Tendenzen wie in Figur 2. Die Kombination 500/60 erhöht, 850/60 verringert die spezifischen Oberflächen sA in $m^2/g$. Interessanter ist der Vergleich der Zahlenpaare aus Masse bezogenem Porenvolumen [cc/g] und dem mittleren Porendurchmesser [QJ Pore nm] für die Meso- und Makroporen aus den Figuren 2 und 3. In der nachfolgenden Tabelle 1 ist dieser Zusammenhang dargestellt:

| Nr. | Bezeichnung | Mesopore | | Makropore | |
|---|---|---|---|---|---|
| | | cc/g | Ø Pore nm | cc/g | Ø Pore nm |
| 1 | MG_18 | - | - | 0,3547 | 208 |
| 2 | MG_18_K(500/60) | 0,2543 | 6,44 | 0,4433 | 194 |
| 3 | MG_18_K(850/60) | - | - | 0,8359 | 159 |
| 4 | MG_18_DSM | - | - | 1,7210 | 165 |
| 5 | MG_18_DSM_K(500/60) | 0,4702 | 5,08 | 3,0710 | 126 |
| 6 | MG_18_DSM_K(850/60) | - | - | 1,5260 | 134 |

Tabelle 1

Für die Herausbildung einer Porenstruktur zur optimalen Phosphatbindung ist der Zerkleinerungsschritt von MG_18, $X_{50}$ ca. 30$\mu$m, auf MG_18_DSM, $X_{50}$ ca. 1$\mu$m, von entscheidender Bedeutung. Durch die intensive mechanische Beanspruchung in der Dampfstrahlmühle wurden Risse im kristallinen Körper erzeugt. Die Makroporen erhöhten sich ca. um den Faktor 5. Durch diese Nanorisse konnten anschließend bei der Thermobehandlung 500°C, 60min, die entstehenden Gase freigesetzt werden. Dabei entstanden die notwendigen Meso- und Makroporen in einem optimalen Verhältnis, was sich im Absorptionsindex von ca. 35 wiederspiegelt. Wird die Behandlungstemperatur auf 850°C erhöht, verschwinden die Mesoporen und es entsteht wieder ein makro-poröses Magnesiumoxid.

In Figur 4 sind die wichtigsten Produktionsschritte und die damit verbundenen Veränderungen nochmals zusammengefasst. Die Kombination aus der Vermahlung von MG_18 mit einer Dampfstrahlmühle auf ca. 1 $\mu$m und die anschließende Aktivierung bei 500°C für 60min erzeugt einen meso-makro-porösen Komplex aus Magnesiumoxid/-carbonat. Dieser zeigt ausgezeichnete Phosphatbindeeigenschaften.

Figur 5 zeigt den Zusammenhang zwischen der maximalen Phosphatbindekapazität $Q_{max}$ und dem Absorptionsindex $AB_{IDX}$. Es gibt eine direkte Proportionalität zwischen beiden Größen. Die Höhe von $AB_{IDX}$ wird entscheidend von der spezifischen Oberfläche und dem intrudierten Volumen der Meso- und der Makroporen bestimmt. Für das optimale Produkt beträgt dieses Verhältnis 783,3 zu 22,2. Das ergibt einen $AB_{IDX}$ von 35,3. Ist der Index hoch, dann ist auch $Q_{max}$ hoch und umgekehrt.

Figur 6 zeigt die Phosphatabsorption in mg pro g Absorber für die verschiedenen Aktivierungen bei einer maximalen Phosphatbelastung von 1140 mg/g. Der Absorber MG_18_DSM_K(500/60) konnte in vitro bei einem pH-Wert >7 947 mg Phosphat pro g binden. Das entspricht 83%. Im Vergleich zum unbehandelten Hydromagnesiten MG_18, $X_{50}$ ca. 30$\mu$m, ist das eine Steigerung um das 11fache. Behandlungstemperaturen kleiner und größer als 500°C verschlechtern die Ergebnisse. Die Behandlungszeit von 60min hatte sich für die verwendete Versuchsapparatur als optimal herausgestellt.

Figur 7 zeigt Modellrechnungen bei einer Phosphatbelastung von 1140 mg/l und die Phosphatbindungen der verschiedenen Absorber bei verschiedenen Absorber-Dosierungen in g/l. Will man z.B. 80% von der Phosphatbelastung binden, dann benötigt man MG_18_DSM_K(500/60) ca. 1g, MG_18_DSM_K(700/60) ca. 3,5g und vom MG_18_K(500/60) ca. 6g. Diese Mengen müssten Patienten im Verhältnis aufnehmen, um vergleichbare Ergebnisse zu erzielen. Deshalb ist eine hohe maximale Phosphatbindekapazität von so großer Bedeutung. Produkte mit einer geringen $Q_{max}$ erreichen auch mit hohen Dosierungen des Absorbers nicht die gewünschten Effekte.

Die vorliegenden Untersuchungen zeigen somit, dass ein mechanisch und thermisch optimal aktivierter Hydromagnesit ein sehr guter Phosphatbinder darstellt. Die Phosphatbindung erfolgt ausschließlich im basischen Milieu. Dazu dient auch die magensaftresistente Ausrüstung mittels Wirbelschichtcoating nach vorhergehender Sprühgefriergranulierung und Gefriertrocknung. Der poröse Komplex aus Magnesiumoxid/-carbonat ist sowohl für die präventive als auch therapeutische Bindung von Nahrungsphosphaten geeignet.

**Patentansprüche**

1. Poröses Magnesiumoxidcarbonat zur Verwendung als Mittel zur Bindung von Phosphaten im Dünndarm mit dem Ziel der Reduzierung von nichtübertragbaren Krankheiten, **gekennzeichnet durch**

   - eine mittlere Teilchengröße zwischen 0,5 und 3μm, bevorzugt zwischen 0,8 und 2μm, insbesondere bevorzugt 1μm;
   - Mesoporen mit einen mittleren Porendurchmesser zwischen 3,5 und 6,5nm, bevorzugt zwischen 4 und 6nm, insbesondere bevorzugt von 5nm, und Makroporen mit einem mittleren Porendurchmesser zwischen 50 und 200nm, bevorzugt zwischen 100 und 150nm, insbesondere bevorzugt von 125nm, jeweils bestimmt mit Quecksilberporosimetrie;
   - wobei die spezifische Oberfläche der Mesoporen zwischen 70 und 90 $m^2/g$, bevorzugt zwischen 75 und 85 $m^2/g$, insbesondere bevorzugt 80 $m^2/g$ und die spezifische Oberfläche der Makroporen zwischen 10 und 14 $m^2/g$, bevorzugt zwischen 11 und 13 $m^2/g$, insbesondere bevorzugt 12 $m^2/g$ beträgt, jeweils bestimmt mit Quecksilberporosimetrie; und
   - einen Absorptionsindex $AB_{IDX}$ für die Bindung von Phosphat zwischen 30 bis 40, bevorzugt von 32 bis 38, insbesondere bevorzugt von 35.

2. Magnesiumoxidcarbonat zur Verwendung nach Anspruch 1, **dadurch gekennzeichnet, dass** es nach der Zerkleinerung einer thermischen Behandlung bei einer Temperatur zwischen 400 und 600°C, bevorzugt zwischen 450 und 550°C, insbesondere bevorzugt bei 500°C über einen Zeitraum von 40 bis 80 min, bevorzugt 50 bis 70 min, insbesondere bevorzugt 60 min, unterzogen wird.

3. Magnesiumoxidcarbonat zur Verwendung nach einem der vorhergehenden Ansprüche, **gekennzeichnet durch** eine maximale Phosphatbindekapazität $Q_{max}$ zwischen 3500 und 4000 mg/g Absorber, bevorzugt zwischen 3700 und 3900 mg/g Absorber.

4. Verfahren zur Herstellung eines Magnesiumoxidcarbonates zur Verwendung nach einem der vorhergehenden Ansprüche, umfassend die Schritte:

   a) Zerkleinerung von Hydromagnesit auf eine mittlere Teilchengröße zwischen 0,5 und 3μm, bevorzugt zwischen 0,8 und 2μm, insbesondere bevorzugt 1μm; und
   b) thermische Behandlung des zerkleinerten Hydromagnesit bei einer Temperatur zwischen 400 und 600°C, bevorzugt zwischen 450 und 550°C, insbesondere bevorzugt bei 500°C über einen Zeitraum von 40 bis 80 min, bevorzugt 50 bis 70 min, insbesondere bevorzugt 60 min.

5. Verfahren zur Herstellung eines Magnesiumoxidcarbonates zur Verwendung nach Anspruch 4, **dadurch gekennzeichnet, dass** das nach der thermischen Behandlung erhaltene poröse Magnesiumoxidcarbonat in einer inerten Gasatmosphäre gelagert wird.

6. Granulat umfassend ein poröses Magnesiumoxidcarbonat zur Verwendung nach einem der Ansprüche 1-3 und eine Zellulosematrix, insbesondere eine Methyl-Zellulose-Matrix.

7. Granulat umfassend ein poröses Magnesiumoxidcarbonat zur Verwendung nach Anspruch 6, **gekennzeichnet durch** ein Masseverhältnis von Zellulose und porösem Magnesiumoxidcarbonat zwischen 0,7:1,3, bevorzugt zwischen 0,9:1,1, insbesondere von 1:1.

8. Granulat umfassend ein poröses Magnesiumoxidcarbonat zur Verwendung nach Anspruch 6 oder 7, **dadurch gekennzeichnet, dass** der Masseanteil des porösen Magnesiumoxidcarbonat als Phosphatabsorber im fertigen Granulat zwischen 40 und 60%, bevorzugt zwischen 45 und 55% und insbesondere bevorzugt bei 50% beträgt.

9. Granulat umfassend ein poröses Magnesiumoxidcarbonat zur Verwendung nach einem der Ansprüche 6 bis 8, **gekennzeichnet durch** einen mittleren Durchmesser zwischen 40 und 450μm, bevorzugt zwischen 250 bis 450μm, insbesondere bevorzugt zwischen 40 und 150μm.

10. Granulat umfassend ein poröses Magnesiumoxidcarbonat zur Verwendung nach einem der Ansprüche 6 bis 9, **gekennzeichnet durch** ein magensaftresistentes Coating.

**11.** Granulat umfassend ein poröses Magnesiumoxidcarbonat zur Verwendung nach Anspruch 10, **gekennzeichnet durch** ein Masseverhältnis von Granulat und Coatingmaterial zwischen 30:1 und 20:1, bevorzugt zwischen 25:1 und 15:1, insbesondere bevorzugt von 10:1.

**12.** Granulat umfassend ein poröses Magnesiumoxidcarbonat zur Verwendung nach einem der Ansprüche 6 bis 11, **dadurch gekennzeichnet, dass** die magensaftresistenten Granulate in Form von Kapseln, Gelen und Suspensionen vorliegen.

**13.** Verfahren zur Herstellung eines Granulates umfassend ein poröses Magnesiumoxidcarbonat zur Verwendung nach einem der Ansprüche 6 bis 11, umfassend die Schritte:

a) Suspension des porösen Magnesiumoxidcarbonates nach einem der Ansprüche 1 bis 3 in eine kolloidale Zellulosedispersion, insbesondere kolloidale Methylzellulosedispersion;
und
b) Granulierung der erhaltenen Suspension mittels Sprüh-Gefrier-Granulierung mit anschließender Gefriertrocknung.

**14.** Verfahren zur Herstellung eines Granulates umfassend ein poröses Magnesiumoxidcarbonat zur Verwendung nach Anspruch 13, **dadurch gekennzeichnet, dass** das Granulat in einem Wirbelschichtapparat mit dem magensaftresistenten Coating beschichtet wird.

**15.** Verfahren zur Herstellung eines Granulates umfassend ein poröses Magnesiumoxidcarbonat zur Verwendung nach einem der Ansprüche 13 bis 14, **dadurch gekennzeichnet, dass** in Schritt a) weitere bioaktive Substanzen in die kolloidale Zellulosedispersion dispergiert werden.

## Claims

**1.** A porous magnesium oxide carbonate for use as an agent for binding phosphates in the small intestine with the aim of reducing non-transmissible diseases, **characterized by**

- a mean particle size between 0.5 and 3 μm, preferably between 0.8 and 2 μm, in particular preferably of 1 μm;
- mesopores with a mean pore diameter between 3.5 and 6.5 nm, preferably between 4 and 6 nm, in particular preferably of 5 nm, and macropores with a mean pore diameter between 50 and 200 nm, preferably between 100 and 150 nm, in particular preferably of 125 nm, each determined by mercury porosimetry;
- wherein the specific surface area of the mesopores lies between 70 and 90 $m^2/g$, preferably between 75 and 85 $m^2/g$, in particular preferably is 80 $m^2/g$, and the specific surface area of the macropores lies between 10 and 14 $m^2/g$, preferably between 11 and 13 $m^2/g$, in particular preferably is 12 $m^2/g$, each determined by mercury porosimetry; and
- an absorption index $AB_{IDX}$ for binding phosphate between 30 and 40, preferably of 32 to 38, in particular preferably of 35.

**2.** The magnesium oxide carbonate for use according to claim 1, **characterized in that** after comminution it is subjected to a thermal treatment at a temperature between 400 and 600°C, preferably between 450 and 550°C, in particular preferably at 500°C over a period of 40 to 80 min, preferably 50 to 70 min, in particular preferably 60 min.

**3.** The magnesium oxide carbonate for use according to any of the preceding claims, **characterized by** a maximum phosphate binding capacity $Q_{max}$ between 3500 and 4000 mg/g of absorber, preferably between 3700 and 3900 mg/g of absorber.

**4.** A method of producing a magnesium oxide carbonate for use according to any of the preceding claims, comprising the following steps:

a) comminution of hydromagnesite to a mean particle size between 0.5 and 3 μm, preferably between 0.8 and 2 μm, in particular preferably of 1 μm; and
b) thermal treatment of the comminuted hydromagnesite at a temperature between 400 and 600°C, preferably between 450 and 550°C, in particular preferably at 500°C over a period of 40 to 80 min, preferably 50 to 70 min, in particular preferably 60 min.

5. The method of producing a magnesium oxide carbonate for use according to claim 4, **characterized in that** the porous magnesium oxide carbonate obtained after the thermal treatment is stored in an inert gas atmosphere.

6. Granules comprising a porous magnesium oxide carbonate for use according to any of claims 1-3 and a cellulose matrix, in particular a methyl cellulose matrix.

7. Granules comprising a porous magnesium oxide carbonate for use according to claim 6, **characterized by** a mass ratio of cellulose and porous magnesium oxide carbonate between 0.7:1.3, preferably between 0.9:1.1, in particular of 1:1.

8. Granules comprising a porous magnesium oxide carbonate for use according to claim 6 or 7, **characterized in that** the mass percentage of the porous magnesium oxide carbonate as a phosphate absorber in the finished granules lies between 40 and 60%, preferably between 45 and 55%, and in particular preferably at 50%.

9. Granules comprising a porous magnesium oxide carbonate for use according to any of claims 6 to 8, **characterized by** a mean diameter between 40 and 450 μm, preferably between 250 and 450 μm, in particular preferably between 40 and 150 μm.

10. Granules comprising a porous magnesium oxide carbonate for use according to any of claims 6 to 9, **characterized by** an enteric coating.

11. Granules comprising a porous magnesium oxide carbonate for use according to claim 10, **characterized by** a mass ratio of granules and coating material between 30:1 and 20:1, preferably between 25:1 and 15:1, in particular preferably of 10:1.

12. Granules comprising a porous magnesium oxide carbonate for use according to any of claims 6 to 11, **characterized in that** the enteric coated granules are present in the form of capsules, gels and suspensions.

13. A method of producing granules comprising a porous magnesium oxide carbonate for use according to any of claims 6 to 11, comprising the following steps:

    a) suspension of the porous magnesium oxide carbonate according to any of claims 1 to 3 in a colloidal cellulose dispersion, in particular a colloidal methyl cellulose dispersion; and
    b) granulation of the obtained suspension by means of spray freeze granulation followed by freeze drying.

14. The method of producing granules comprising a porous magnesium oxide carbonate for use according to claim 13, **characterized in that** the granules are coated with the enteric coating in a fluidized bed apparatus.

15. The method of producing granules comprising a porous magnesium oxide carbonate for use according to any of claims 13 to 14, **characterized in that** in step a) further bioactive substances are dispersed into the colloidal cellulose dispersion.

## Revendications

1. Carbonate d'oxyde de magnésium poreux destiné à l'utilisation comme agent de liaison de phosphates dans l'intestin grêle dans le but de réduire les maladies non transmissibles,
   **caractérisé par**

    - une taille moyenne de particules comprise entre 0,5 et 3 μm, de préférence entre 0,8 et 2 μm, en particulier de préférence de 1 μm ;
    - mésopores ayant un diamètre moyen de pores compris entre 3,5 et 6,5 nm, de préférence entre 4 et 6 nm, en particulier de préférence de 5 nm, et macropores ayant un diamètre moyen de pores compris entre 50 et 200 nm, de préférence entre 100 et 150 nm, en particulier de préférence de 125 nm, déterminés chacun par porosimétrie au mercure ;
    - la surface spécifique des mésopores étant comprise entre 70 et 90 $m^2/g$, de préférence entre 75 et 85 $m^2/g$, en particulier de préférence étant 80 $m^2/g$, et la surface spécifique des macropores étant comprise entre 10 et 14 $m^2/g$, de préférence entre 11 et 13 $m^2/g$, en particulier de préférence étant 12 $m^2/g$, chacune déterminée

par porosimétrie au mercure ; et
- un indice d'absorption $AB_{IDX}$ pour la liaison de phosphate compris entre 30 et 40, de préférence entre 32 et 38, en particulier de préférence de 35.

**2.** Carbonate d'oxyde de magnésium destiné à l'utilisation selon la revendication 1, **caractérisé en ce que**, après le broyage, il est soumis à un traitement thermique à une température comprise entre 400 et 600°C, de préférence entre 450 et 550°C, en particulier de préférence à 500°C, pendant une durée de 40 à 80 minutes, de préférence de 50 à 70 minutes, en particulier de préférence de 60 minutes.

**3.** Carbonate d'oxyde de magnésium destiné à l'utilisation selon l'une quelconque des revendications précédentes, **caractérisé par** une capacité maximale de liaison de phosphate $Q_{max}$ comprise entre 3500 et 4000 mg/g d'absorbant, de préférence entre 3700 et 3900 mg/g d'absorbant.

**4.** Procédé de préparation d'un carbonate d'oxyde de magnésium destiné à l'utilisation selon l'une quelconque des revendications précédentes, comprenant les étapes suivantes :

a) broyage d'hydromagnésite à une taille moyenne de particules comprise entre 0,5 et 3 μm, de préférence entre 0,8 et 2 μm, en particulier de préférence de 1 μm ; et
b) traitement thermique de l'hydromagnésite broyée à une température comprise entre 400 et 600°C, de préférence entre 450 et 550°C, en particulier de préférence à 500°C, pendant une durée de 40 à 80 minutes, de préférence de 50 à 70 minutes, en particulier de préférence de 60 minutes.

**5.** Procédé de préparation d'un carbonate d'oxyde de magnésium destiné à l'utilisation selon la revendication 4, **caractérisé en ce que** le carbonate d'oxyde de magnésium poreux obtenu après le traitement thermique est stocké dans une atmosphère de gaz inerte.

**6.** Granulat comprenant un carbonate d'oxyde de magnésium poreux destiné à l'utilisation selon l'une quelconque des revendications 1 à 3 et une matrice de cellulose, en particulier une matrice de méthylcellulose.

**7.** Granulat comprenant un carbonate d'oxyde de magnésium poreux destiné à l'utilisation selon la revendication 6, **caractérisés par** un rapport en masse de cellulose et de carbonate d'oxyde de magnésium poreux compris entre 0,7:1,3, de préférence entre 0,9:1,1, en particulier de 1:1.

**8.** Granulat comprenant un carbonate d'oxyde de magnésium poreux destiné à l'utilisation selon la revendication 6 ou 7, **caractérisé en ce que** le pourcentage en masse du carbonate d'oxyde de magnésium poreux comme absorbeur de phosphate dans le granulat fini est comprise entre 40 et 60%, de préférence entre 45 et 55% et en particulier de préférence à 50%.

**9.** Granulat comprenant un carbonate d'oxyde de magnésium poreux destiné à l'utilisation selon l'une quelconque des revendications 6 à 8, **caractérisé par** un diamètre moyen compris entre 40 et 450 μm, de préférence entre 250 et 450 μm, en particulier de préférence entre 40 et 150 μm.

**10.** Granulat comprenant un carbonate d'oxyde de magnésium poreux destiné à l'utilisation selon l'une quelconque des revendications 6 à 9, **caractérisé par** un enrobage entérique.

**11.** Granulat comprenant un carbonate d'oxyde de magnésium poreux destiné à l'utilisation selon la revendication 10, **caractérisé par** un rapport en masse de granulat et matériau d'enrobage comprise entre 30:1 et 20:1, de préférence entre 25:1 et 15:1, en particulier de préférence de 10:1.

**12.** Granulat comprenant un carbonate d'oxyde de magnésium poreux destiné à l'utilisation selon l'une quelconque des revendications 6 à 11, **caractérisé en ce que** le granulat gastro-résistant est présent sous forme de capsules, de gels et de suspensions.

**13.** Procédé de préparation d'un granulat comprenant un carbonate d'oxyde de magnésium poreux destiné à l'utilisation selon l'une quelconque des revendications 6 à 11, comprenant les étapes suivantes :

a) suspension du carbonate d'oxyde de magnésium poreux selon l'une quelconque des revendications 1 à 3 dans une dispersion de cellulose colloïdale, en particulier une dispersion de méthylcellulose colloïdale ; et

b) granulation de la suspension obtenue par granulation par pulvérisation et lyophilisation, puis lyophilisation et séchage.

**14.** Procédé de préparation d'un granulat comprenant un carbonate d'oxyde de magnésium poreux destiné à l'utilisation selon la revendication 13, **caractérisé en ce que** le granulat est revêtu de l'enrobage entérique dans un appareil à lit fluidisé.

**15.** Procédé de préparation d'un granulat comprenant un carbonate d'oxyde de magnésium poreux destiné à l'utilisation selon l'une quelconque des revendications 13 à 14, **caractérisé en ce que**, dans l'étape a), d'autres substances bioactives sont dispersées dans la dispersion de cellulose colloïdale.

Figur 1

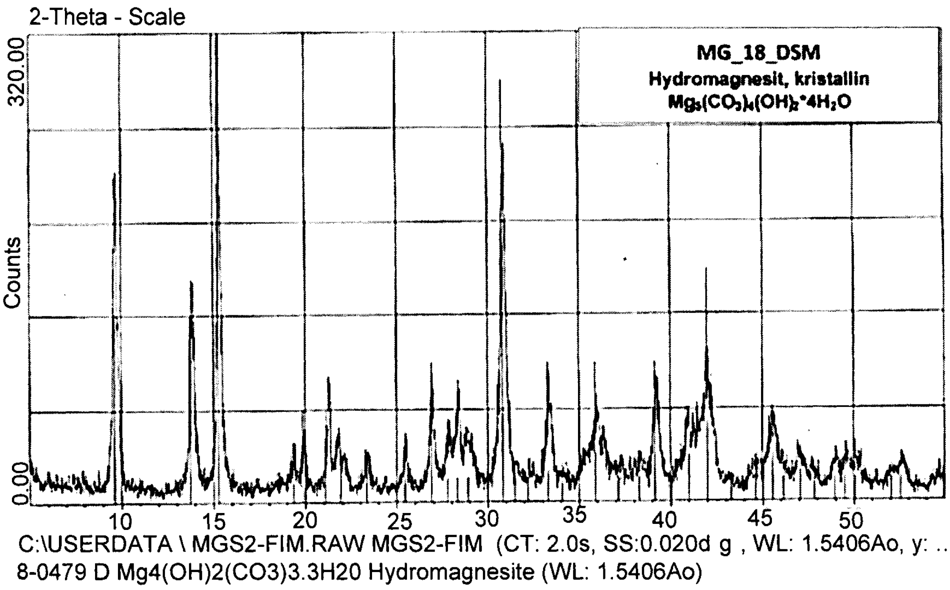


C:\USERDATA \ MGS2-FIM.RAW MGS2-FIM (CT: 2.0s, SS:0.020d g , WL: 1.5406Ao, y: .......)
8-0479 D Mg4(OH)2(CO3)3.3H20 Hydromagnesite (WL: 1.5406Ao)

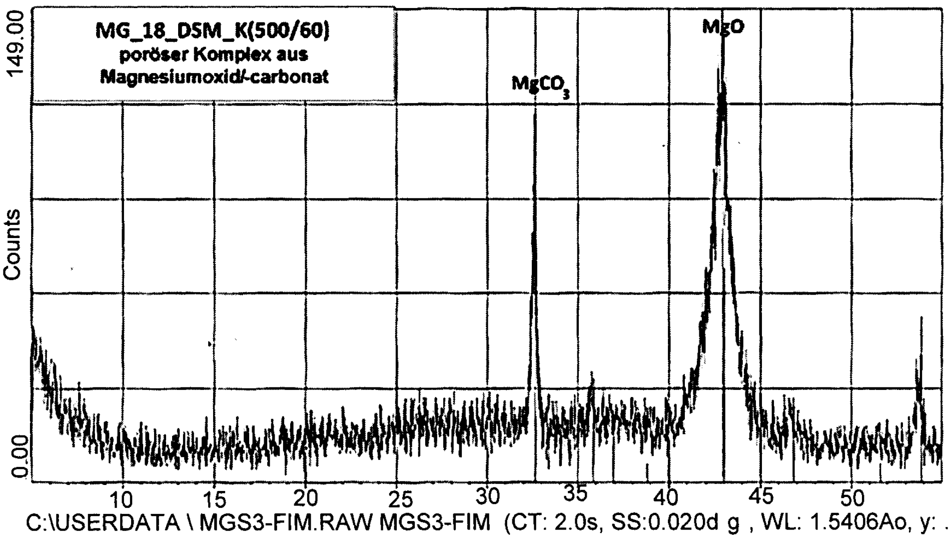


C:\USERDATA \ MGS3-FIM.RAW MGS3-FIM (CT: 2.0s, SS:0.020d g , WL: 1.5406Ao, y: .......)
8-0479 MgCO3 Magnesite. Syn (WL: 1.5406Ao)
4-0829 MgO Per:close. syn (WL: 1.5406Ao)

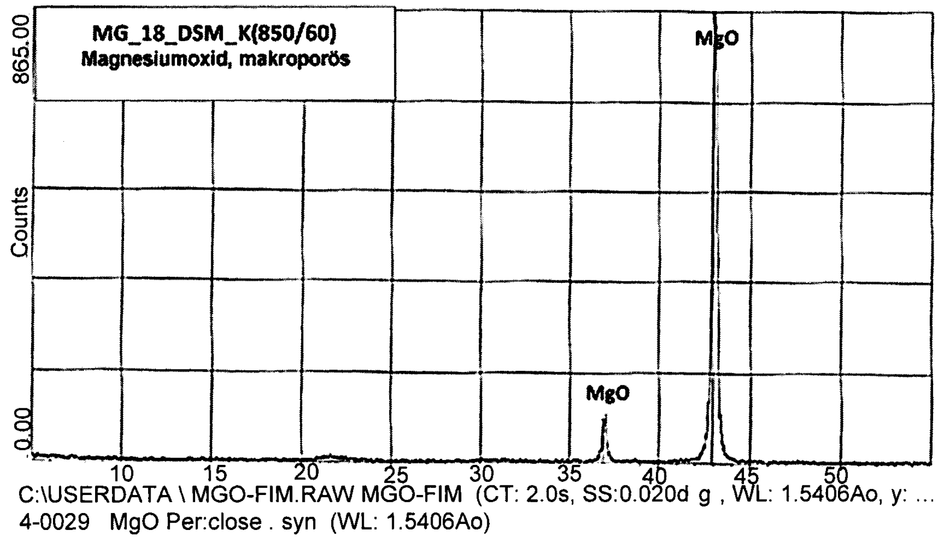


C:\USERDATA \ MGO-FIM.RAW MGO-FIM (CT: 2.0s, SS:0.020d g , WL: 1.5406Ao, y: .......)
4-0029 MgO Per:close . syn (WL: 1.5406Ao)

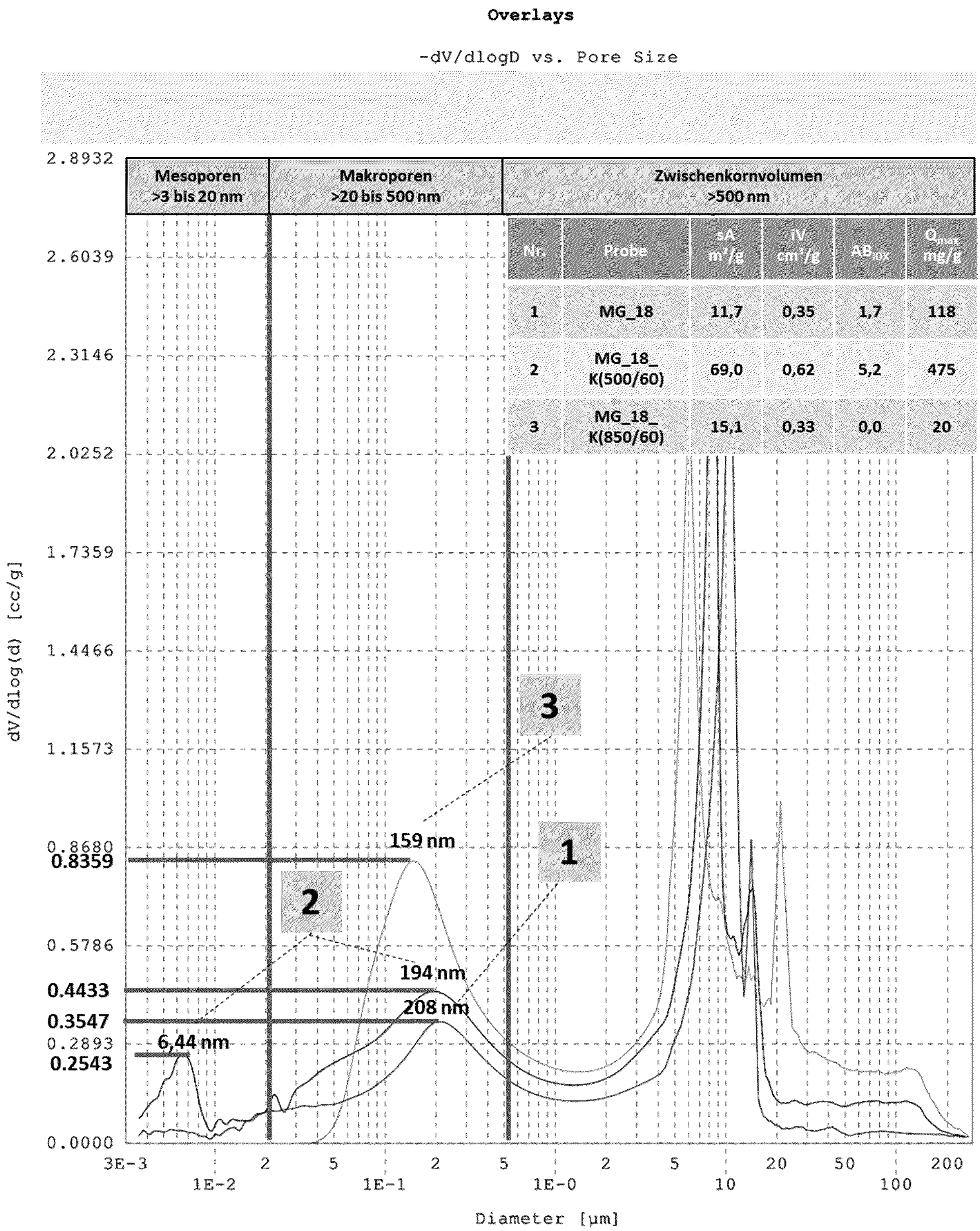
Overlays
-dV/dlogD vs. Pore Size
Mesoporen >3 bis 20 nm
Makroporen >20 bis 500 nm
Zwischenkornvolumen >500 nm
Nr. | Probe | sA m²/g | iV cm³/g | AB IDX | Q max mg/g
1 | MG_18 | 11,7 | 0,35 | 1,7 | 118
2 | MG_18_K(500/60) | 69,0 | 0,62 | 5,2 | 475
3 | MG_18_K(850/60) | 15,1 | 0,33 | 0,0 | 20
2.8932
2.6039
2.3146
2.0252
1.7359
1.4466
1.1573
0.8680
0.8359
0.5786
0.4433
0.3547
0.2893
0.2543
0.0000
dV/dlog(d) [cc/g]
159 nm
194 nm
208 nm
6,44 nm
1
2
3
3E-3
2
5
2
5
2
5
20
50
200
1E-2
1E-1
1E-0
10
100
Diameter [µm]

Figur 2

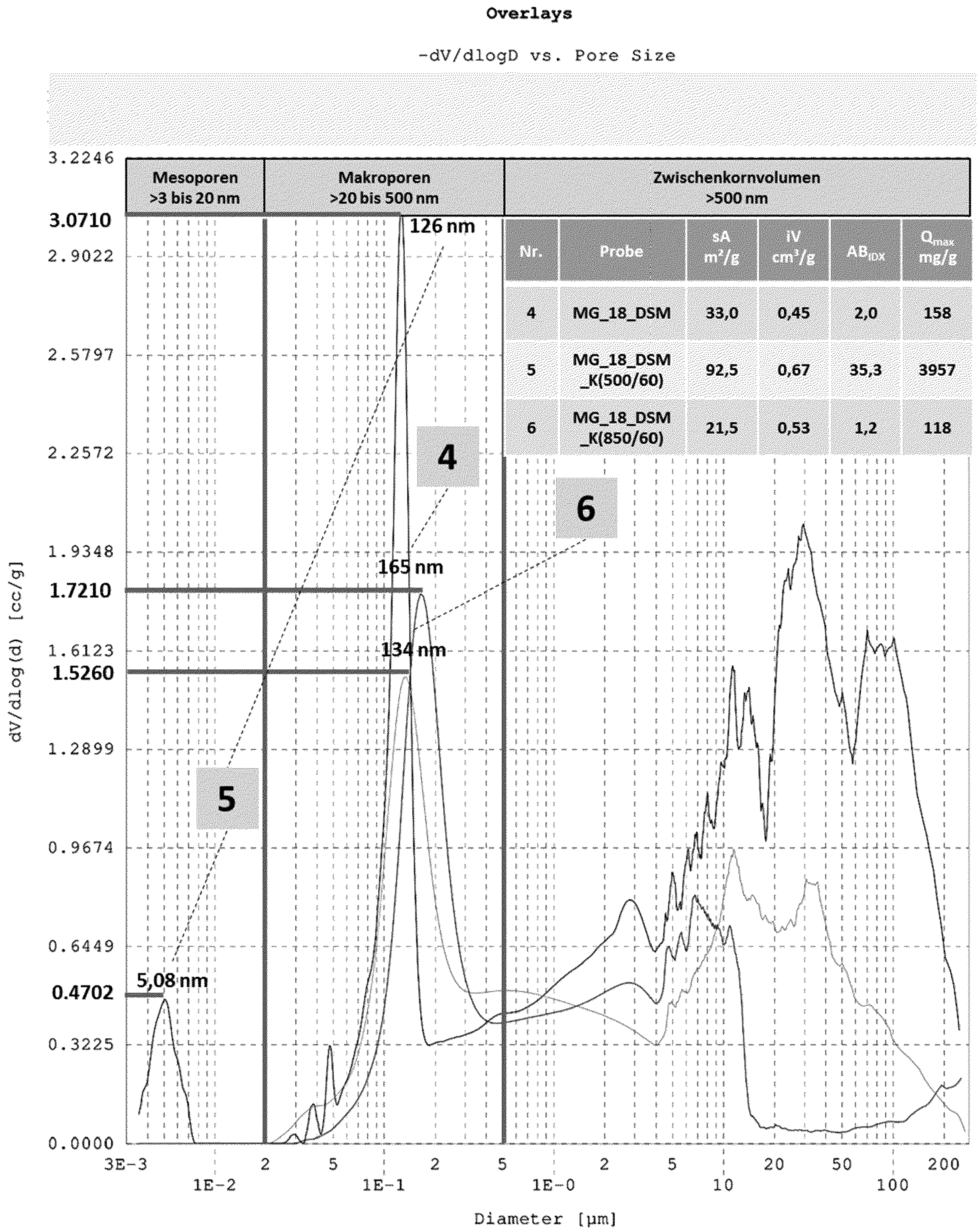


| Nr. | Probe | sA $m^2/g$ | iV $cm^3/g$ | $AB_{IDX}$ | $Q_{max}$ mg/g |
|---|---|---|---|---|---|
| 4 | MG_18_DSM | 33,0 | 0,45 | 2,0 | 158 |
| 5 | MG_18_DSM _K(500/60) | 92,5 | 0,67 | 35,3 | 3957 |
| 6 | MG_18_DSM _K(850/60) | 21,5 | 0,53 | 1,2 | 118 |

# Figur 3

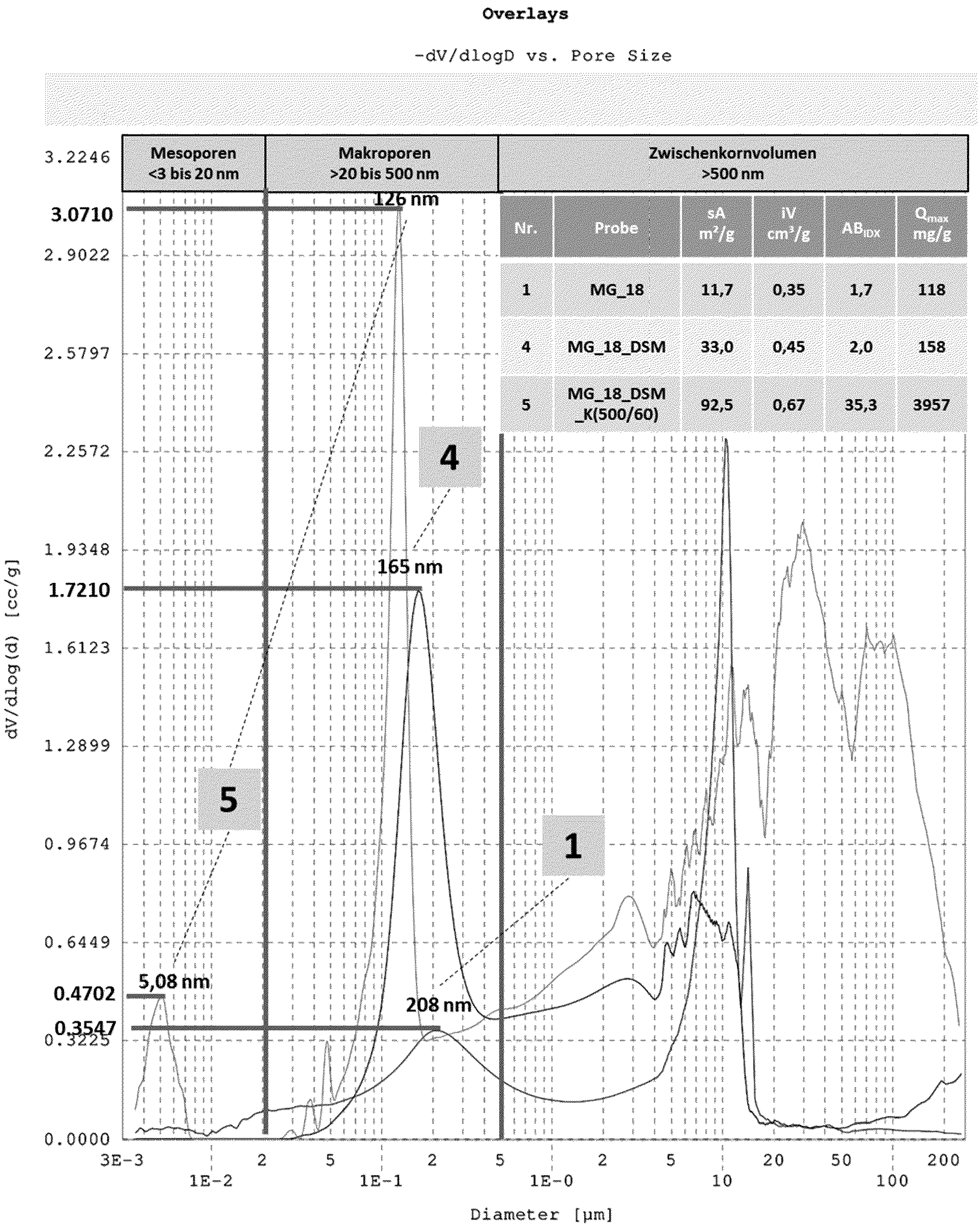


| Nr. | Probe | sA $m^2/g$ | iV $cm^3/g$ | $AB_{IDX}$ | $Q_{max}$ mg/g |
|---|---|---|---|---|---|
| 1 | MG_18 | 11,7 | 0,35 | 1,7 | 118 |
| 4 | MG_18_DSM | 33,0 | 0,45 | 2,0 | 158 |
| 5 | MG_18_DSM_K(500/60) | 92,5 | 0,67 | 35,3 | 3957 |

**Figur 4**

**Zusammenhang zwischen der max. Phosphatbindekapazität $Q_{max}$ und dem Absorptionsindex $AB_{IDX}$**

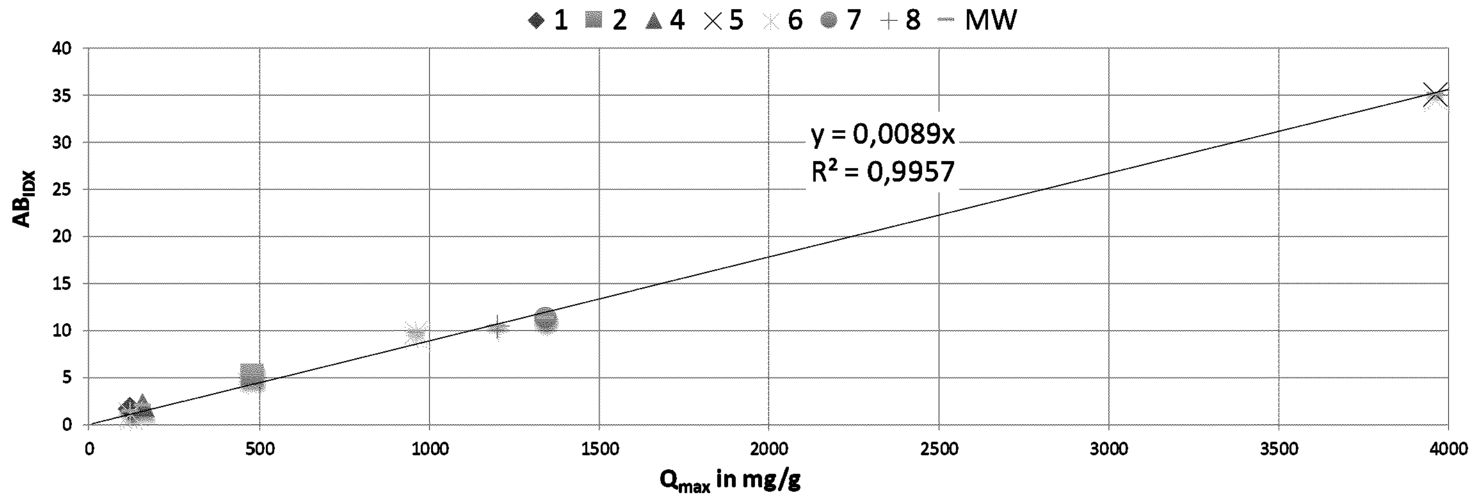


| Produkt | | Mesoporen >3 bis 20 nm | | | | Makroporen >20 bis 500 nm | | | | Gesamt | | | |
|---|---|---|---|---|---|---|---|---|---|---|---|---|---|
| Nr. | Bezeichnung | Ø Pore | $IV_{MEP}$ | $sA_{MEP}$ | $sA_{MEP}/IV_{MEP}$ | Ø Pore | $IV_{MAP}$ | $sA_{MAP}$ | $sA_{MAP}/IV_{MAP}$ | sA | IV | $AB_{IDX}$ | $Q_{max}$ |
| | | nm | $cm^3/g$ | $m^2/g$ | $m^2/cm^3$ | nm | $cm^3/g$ | $m^2/g$ | $m^2/cm^3$ | $m^2/g$ | $cm^3/g$ | $AV_{MEP}/AV_{MAP}$ | mg/g |
| 1 | MG_18 | / | 0,0711 | 3,50 | 49,2 | 217,0 | 0,2826 | 8,20 | 29,0 | 11,70 | 0,35 | 1,7 | 118 |
| 2 | MG_18_K(500/60) | 6,06 | 0,0832 | 30,70 | 369,1 | 186,0 | 0,5400 | 38,30 | 70,9 | 69,00 | 0,62 | 5,2 | 475 |
| 4 | MG_18_DSM | 4,80 | 0,0289 | 4,10 | 141,9 | 171,0 | 0,4172 | 28,90 | 69,3 | 33,00 | 0,45 | 2,0 | 158 |
| 5 | MG_18_DSM_K(500/60) | 5,04 | 0,1020 | 79,90 | 783,3 | 115,0 | 0,5670 | 12,60 | 22,2 | 92,50 | 0,67 | 35,3 | 3957 |
| 6 | MG_18_DSM_K(850/60) | / | 0,0326 | 1,63 | 50,1 | 134,5 | 0,4944 | 19,90 | 40,3 | 21,53 | 0,53 | 1,2 | 118 |
| 7 | MG_18_DSM_K(700/60) | 9,15 | 0,0905 | 44,90 | 496,1 | 89,2 | 0,5130 | 22,56 | 44,0 | 67,46 | 0,60 | 11,3 | 1337 |
| 8 | MG_18_DSM_K(500-700/60) | 6,67 | 0,1593 | 90,60 | 568,7 | 76,0 | 0,4957 | 27,10 | 54,7 | 117,70 | 0,66 | 10,4 | 1202 |

**Figur 5**

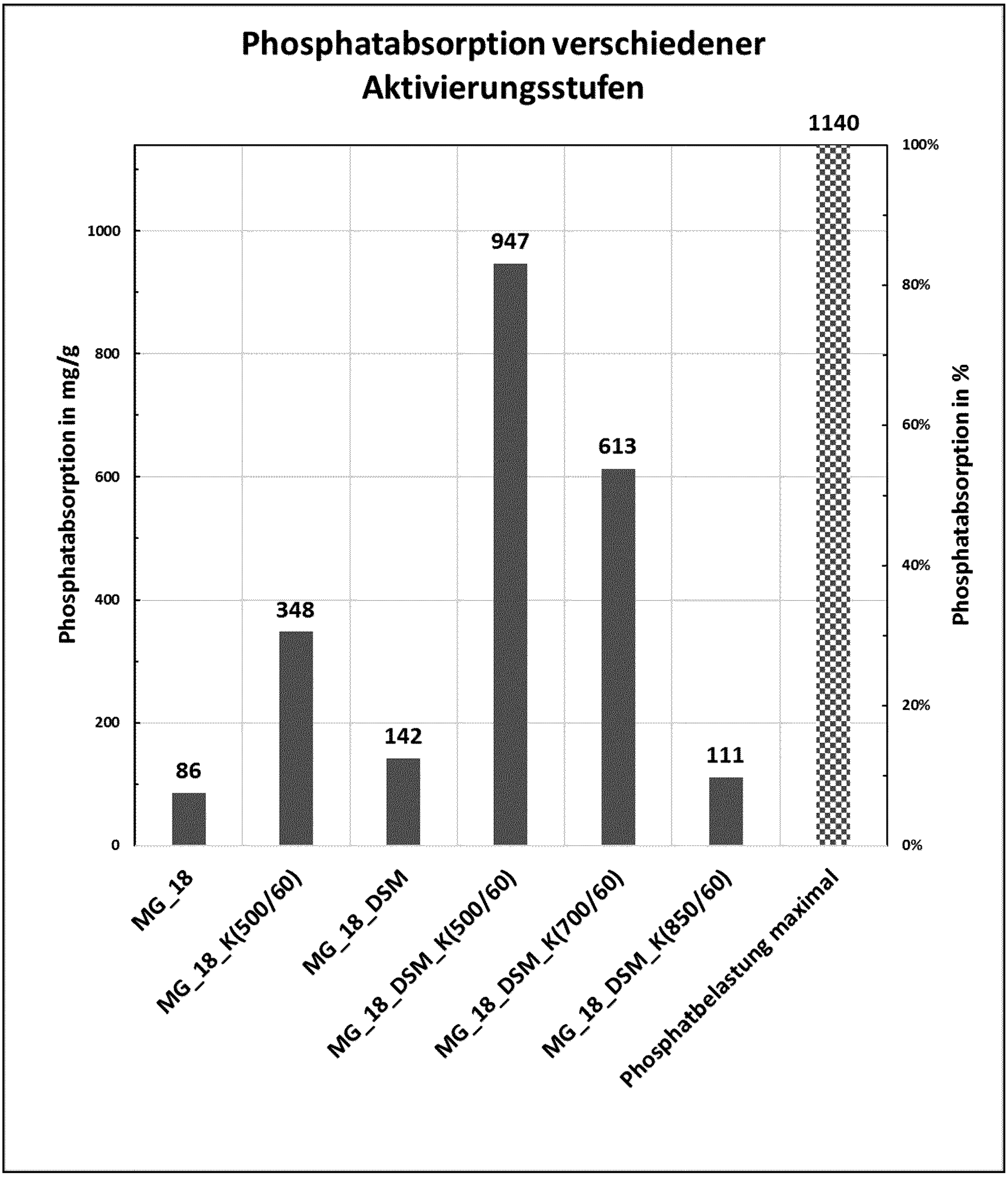
Phosphatabsorption verschiedener Aktivierungsstufen
Phosphatabsorption in mg/g
Phosphatabsorption in %
0
200
400
600
800
1000
0%
20%
40%
60%
80%
100%
86
348
142
947
613
111
1140
MG_18
MG_18_K(500/60)
MG_18_DSM
MG_18_DSM_K(500/60)
MG_18_DSM_K(700/60)
MG_18_DSM_K(850/60)
Phosphatbelastung maximal

Figur 6

## Phosphatbindung nach verschiedenen Aktivierungen

**Q = (Qmax*K*c)/(1+K*c)** Molmasse µg/µmol: 94,97

**Belastung:** 12000 µmol/l

1140 mg/l

| Absorber g/l | Phosphat µmol/l | c µmol/g Abs | MG_18 | MG_18_K(500/60) | MG_18_DSM | MG_18_DSM_K(500/60) | MG_18_DSM_K(850/60) | MG_18_DSM_K(700/60) |
|---|---|---|---|---|---|---|---|---|
| **0,500** | 12000,00 | 24000,00 | 4% | 18% | 7% | 70% | 5% | 37% |
| **1,000** | 12000,00 | 12000,00 | 8% | 31% | 12% | 82% | 10% | 54% |
| **1,500** | 12000,00 | 8000,00 | 10% | 40% | 18% | 87% | 14% | 63% |
| **2,000** | 12000,00 | 6000,00 | 12% | 48% | 23% | 90% | 18% | 70% |
| **2,500** | 12000,00 | 4800,00 | 13% | 55% | 27% | 92% | 23% | 74% |
| **3,000** | 12000,00 | 4000,00 | 15% | 60% | 31% | 93% | 26% | 77% |
| **3,500** | 12000,00 | 3428,57 | 16% | 64% | 35% | 94% | 30% | 80% |
| **4,000** | 12000,00 | 3000,00 | 17% | 68% | 38% | 95% | 33% | 82% |
| **4,500** | 12000,00 | 2666,67 | 17% | 71% | 41% | 95% | 37% | 84% |
| **5,000** | 12000,00 | 2400,00 | 18% | 74% | 44% | 96% | 40% | 85% |
| **5,500** | 12000,00 | 2181,82 | 19% | 77% | 47% | 96% | 43% | 86% |
| **6,000** | 12000,00 | 2000,00 | 19% | 79% | 49% | 97% | 46% | 87% |

## Phosphat-Absorption bei 12000 µmol/l (1140 mg/l)

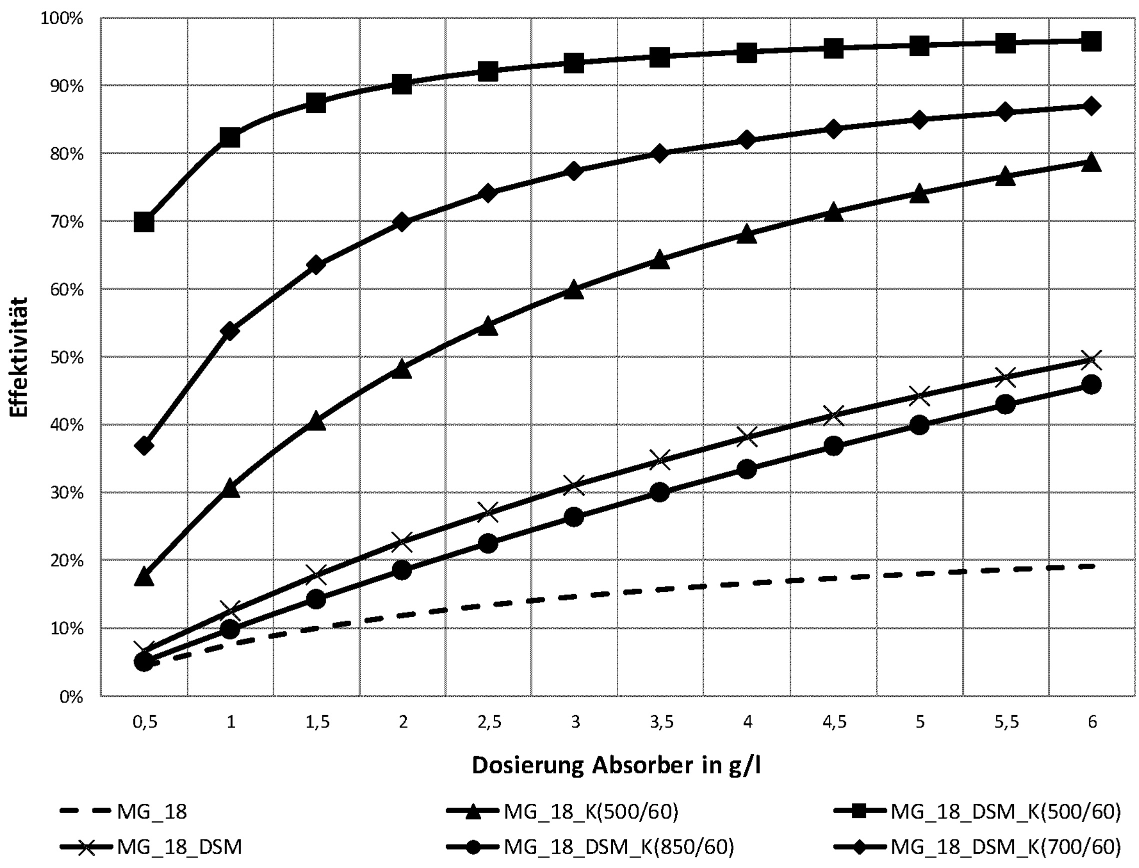


**Figur 7**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102012209411
- EP 2692691 A1


### In der Beschreibung aufgeführte Nicht-Patentliteratur


- **VON BARNET et al.** Epidemiology of multimorbodity and implications for health care, research, and medical education: a cross-sectional study. *Lancet,* 2012, vol. 380, 37-43
- **VON S. LICHTER et al.** Lifetime risk and multimorbidity of non-communicable diseases and disease-free life expectancy in the general population: A population-based cohort study. *PLos Med,* 2019, 16
- **ZOCCALI et al.** The systemic nature of CKD. *Nat Rev Nephrol.,* 2017, vol. 13, 344-358
- **MAKOTO KURO-O et al.** Mutation of the mouse klotho gene leads to a syndrome resembling ageing. *Nature,* 1997, vol. 390, 45-51
- **KUROSU et al.** *J.Biol. Chem.,* 2006, vol. 281, 6120-3
- **VON MAKATO KURU-O et al.** Molecular Mechanisms Underlying Accelerated Aging by Defects in the FGF23-Klotho System. *Int. J. Nephrology,* 2018
- **EDWARD R. SMITH et al.** Biochemical transformation of calciprotein particles in uraemia. *Bone,* Mai 2018, vol. 110, 355-367
- **YUTAKA MIURA et al.** Identification and quantification of plasma calciprotein particles with distinct physical properties in patiens with chronic kidney disease. *Scientific Reports,* 2018, 8
- **PAVIC et al.** *Secreted Klotho and FGF23 in chronic kidney disease Stage,* 1-5
- *Nephrol. Dial. Transplant,* 2013, vol. 28, 352-359
- **SPEER et al.** A single preoperativ FGF23 measurement is a strong predictor of outcome in patients undergoing elective cardiac surgery: a prospective observational study. *Crit Care,* 2015, vol. 19, 190
- **Al KHODOR et al.** Gut microbiome and kidney disease: a bidirectional relationship. *Pediatr Nephrol,* 2017, vol. 32, 921-931
- **PUELLES et al.** Glomerular number and size variability and risk for kidney disease. *Curr. Opin. Nephrol. Hypertens.,* 2011, vol. 20, 7-15
- **HUGHSON et al.** Glomerular number and size in autopsy kidneys: The relationship to birth weight. *Kindney Int.,* 2003, vol. 63, 2113-22
- **GURUSINGHE et al.** Developmental Origins and Nephron Endowment in Hypertension. *Front Pediatr,* 2017, vol. 5, 151
- **HAUT et al.** Renal toxicity of phosphate in rats. *Kidney Int.,* 1980, vol. 17, 722-31
- **SUETONIA C. PALMER et al.** Phosphate-Binding Agents in Adults With CKD: A Network Meta-analaysis of Randomized Trials. *Am J Kidney Dis.,* November 2016, vol. 68 (5), 691-702
- **W.B. WHITE.** *Environmental Geology,* 1997, vol. 30, 46-58